# EUROPEAN PATENT APPLICATION

(11) **EP 4 789 659 A1**
(43) Date of publication of application: **12.08.2026**
(21) Application number: 24874715.6
(22) Date of filing: 04.10.2024
(51) Int. Cl.: A61K 9/14, A61K 31/7088, A61K 35/12, A61K 38/00, A61K 39/395, A61K 45/00, A61K 47/12, A61K 47/24, C07K 2/00, C07K 16/00, C12N 5/00, C12N 5/10, C12N 15/11

(54) **METHOD FOR INTRODUCING TARGET MOLECULE INTO LIPID VESICLE AND CELL, AND ANTIBODY DISPERSION PREPARATION**

(30) Priority: 06.10.2023 JP 2023174618; 29.11.2023 JP 2023201394
(71) Applicant: Novigo Pharma, Inc., Fukuoka-shi, Fukuoka 819-0388 (JP)
(72) Inventor: CUI LIN, Fukuoka-shi, Fukuoka 819-0388 (JP); KITAOKA Momoko, Fukuoka-shi, Fukuoka 819-0388 (JP); ISHIHAMA Kohei, Fukuoka-shi, Fukuoka 819-0388 (JP)
(74) Representative: Hoffmann Eitle
(86) International application number: PCT/JP2024/035539
(87) International publication number: WO 2025/075115

(57) **Abstract**

The present disclosure provides a method for introducing molecules of interest into lipid vesicles (e.g., extracellular vesicles). The present disclosure provides lipid vesicles (e.g., extracellular vesicles) obtained using the method for introducing molecules of interest into lipid vesicles (e.g., extracellular vesicles). According to the present disclosure, a complex (first complex) of a lipid (e.g., an ionic lipid, particularly a liquid lipid composition) and a molecule of interest fuses with a lipid vesicle (e.g., extracellular vesicle) to form a complex (second complex). The first complex and the second complex are suitable for introducing molecules of interest into cells, and the second complex is particularly suitable for introducing molecules of interest into cells. The molecule of interest may be a protein (e.g., an antibody), a nucleic acid, or another compound.

## Description

### TECHNICAL FIELD

The present disclosure relates to methods for introducing molecules of interest into lipid vesicles and cells. The present disclosure relates to lipid vesicles obtained using the method for introducing molecules of interest into lipid vesicles. The present disclosure also relates to a dispersion formulation comprising a complex comprising an antibody and a lipid, and a base, wherein the complex is dispersed in the base.

### BACKGROUND ART

Drug delivery systems enable treatment of diseases while avoiding serious adverse events by delivering a therapeutic agent in the required amount to the required site, and are expected to be deployed for treating a wide variety of diseases. In particular, nanoparticles such as liposomes and lipid nanoparticles (LNPs) can permeate through intercellular spaces and membranes due to their small size, and can also be modified on their surfaces with antibodies, polyethylene glycol (PEG), or the like, imparted with environmental responsiveness, or used to protect drugs from the surrounding environment in the body, and thus their application to drug delivery systems is being advanced. Furthermore, nano-sized particles can also utilize the EPR effect (Enhanced Permeability and Retention effect) targeting tumor tissues.

Liposome formulations are vesicles having a lipid bilayer structure composed of phospholipids, and are capable of encapsulating water-soluble agents in their interior and hydrophobic drugs within the lipid bilayer. Their development has been most widely advanced, and currently, in Japan, antitumor agents (Onivyde, Doxil), antifungal agents (AmBisome), and therapeutic agents for age-related macular degeneration (Visudyne) are also used as pharmaceuticals. Lipid nanoparticles are nanoparticles usually composed of a plurality of lipids having different functions, and are used by encapsulating water-soluble agents in the internal hydrophilic region and oil-soluble agents in the hydrophobic region. In COVID-19 vaccines, lipid nanoparticles encapsulating mRNA (Patent Documents 1 and 2) are used as pharmaceuticals (Comirnaty, Spikevax).

In recent years, development has been advanced to utilize extracellular vesicles (e.g., exosomes) released by cells as novel drug delivery carriers having high biocompatibility and low immunogenicity. Exosomes are nano-sized secretory vesicles (30 to 200 nm) produced from all cells and present throughout the body, and are present in all organisms. Exosomes have a lipid bilayer structure, encapsulate proteins and nucleic acids on the inside, and display signal molecules on their surface, and are considered to function in transmitting signals and molecules between cells in the body. Tsai et al. reported that exosomes have lower cytotoxicity to cells compared to LNPs, and that exosomes loaded with mRNA of the spike protein and nucleocapsid protein of SARS-CoV-2 elicited high antibody production levels *in vivo* in mice (Non-Patent Document 1). However, with exosomes alone, the types and amounts of drugs that can be loaded may be limited depending on the state of surface molecules and charge state. Therefore, there are also reports of fusing exosomes and liposomes for use as drug delivery carriers. Hybrid particles of exosomes and liposomes not only overcome the above-mentioned problems when using exosomes alone, but also facilitate enhancing the stability of particles and performing surface modification. Lv et al. reported a method of effectively inhibiting tumorigenesis by combining drug delivery using hybrid particles of exosomes and liposomes with hyperthermic intraperitoneal chemotherapy (Non-Patent Document 2).

### PRIOR ART DOCUMENTS

### PATENT DOCUMENTS

[Patent Document 1] US 8,058,069 B
[Patent Document 2] US 8,278,036 B

### NON-PATENT DOCUMENTS

[Non-Patent Document 1] J Biol Chem. 2021 297(5):101266
[Non-Patent Document 2] Adv Sci 2020 29;7(18):2000515

### SUMMARY OF INVENTION

The present disclosure provides a method for introducing molecules of interest into lipid vesicles (e.g., extracellular vesicles). The present disclosure provides lipid vesicles (e.g., extracellular vesicles) obtained using the method for introducing molecules of interest into lipid vesicles (e.g., extracellular vesicles). The present disclosure also provides a dispersion formulation comprising a complex comprising an antibody and a lipid, and a base, wherein the complex is dispersed in the base.

In the present disclosure, it was found that, for example, a complex (first complex) of an ionic lipid (particularly a natural or non-natural cationic lipid) or a nonionic lipid (particularly a lipid comprising a non-natural lipid, particularly a non-natural nonionic lipid) and a molecule of interest can be obtained, and a hybrid particle (second complex) of the first complex and a lipid vesicle (e.g., extracellular vesicle) or exosome can be formed. In the present disclosure, it was also found that the first complex and the second complex are useful as drug delivery carriers loaded with exogenous molecules such as nucleic acids and proteins (e.g., antibodies, enzymes, etc.). The lipid constituting the first complex is an additional lipid relative to the lipid constituting the lipid vesicle (e.g., extracellular vesicle) or exosome in the second complex, and preferably differs from any of the lipids constituting the lipid vesicle (e.g., extracellular vesicle) or exosome, and is, for example, a non-natural lipid.

According to the present disclosure, the following inventions may be provided.
(1) A composition comprising a complex of a molecule of interest (e.g., a water-soluble molecule) and a lipid mixture (e.g., a mixture of one or two or more lipids, or, e.g., a natural lipid or a non-natural lipid, e.g., comprising a lipid vesicle or a lipid nanoparticle) and a lipid vesicle (preferably, an extracellular vesicle).
(2) The composition according to (1) above, wherein the lipid mixture and the lipid vesicle (e.g., extracellular vesicle) form a vesicle, and the molecule of interest is encapsulated in the formed vesicle.
(3) The composition according to (1) or (2) above, wherein the lipid mixture comprises a cationic lipid (e.g., 1,2-dimyristoyl-sn-glycero-3-ethylphosphatidylcholine (EDMPC⁺)) or a salt thereof.
(4) The composition according to (3) above, comprising a cationic lipid and an anionic lipid, preferably, the cationic lipid and the anionic lipid are lipids that become liquid at atmospheric pressure and room temperature when mixed at a 1:1 charge ratio.
(5) The composition according to (3) or (4) above, wherein the anionic lipid is represented by R-COO⁻, wherein R represents a substituted or unsubstituted alkyl group, or a substituted or unsubstituted alkenyl group, at least one ethylene group constituting the alkenyl group may be substituted with a vinylene group, and preferably R is a saturated fatty acid or a cis-type unsaturated fatty acid.
(6) The composition according to any one of (3) to (5) above, wherein the cationic lipid is represented by R-O-P(OR¹)(OR²)(=O), wherein R represents a substituted or unsubstituted alkyl group, or a substituted or unsubstituted alkenyl group, at least one ethylene group constituting the alkenyl group may be substituted with a vinylene group, and preferably R is a cis-type unsaturated fatty acid, R¹ represents an alkyl group substituted with a cationic group, and R² represents a hydrogen atom or a substituted or unsubstituted alkyl group.
(7) The composition according to any one of (1) to (6) above, wherein the molecule of interest is a polypeptide.
(8) The composition according to any one of (1) to (6) above, wherein the molecule of interest is a nucleic acid.
(9) The composition according to any one of (1) to (6) above, wherein the molecule of interest is an antibody or an antigen-binding fragment thereof.
(10) The composition according to any one of (1) to (9) above, for delivering a molecule of interest to a cell.
(11) The composition according to any one of (1) to (10) above, wherein the complex is obtained by a method comprising: obtaining a complex (first complex) of a molecule of interest and a lipid mixture; and contacting the obtained complex with a lipid vesicle (e.g., extracellular vesicle) to obtain a composite particle (second complex) of the complex and the lipid vesicle (e.g., extracellular vesicle), wherein the molecule of interest is encapsulated in the lipid vesicle (e.g., extracellular vesicle).
(12) A method for introducing a molecule of interest into a lipid vesicle (e.g., extracellular vesicle), comprising: obtaining a complex (first complex) of a molecule of interest and a lipid mixture; and contacting the obtained complex with a lipid vesicle (e.g., extracellular vesicle) to obtain a composite particle (second complex) of the complex and the lipid vesicle (e.g., extracellular vesicle), whereby the molecule of interest is introduced into the composite particle.
(13) A method for introducing a molecule of interest into a cell, comprising: obtaining a complex (first complex) of a molecule of interest and a lipid mixture; contacting the obtained complex with a lipid vesicle (e.g., extracellular vesicle) to obtain a composite particle (second complex) of the complex and the lipid vesicle (e.g., extracellular vesicle), whereby the molecule of interest is introduced into the composite particle; and contacting the obtained composite particle with a cell to introduce the molecule of interest into the cell.
(21) The composition or method according to any one of the above, wherein the lipid mixture comprises a cationic lipid.
(22) The composition or method according to (21) above, wherein the cationic lipid comprises a non-charged phosphate ester moiety and a quaternary ammonium cation.
(23) The composition or method according to (21) or (22) above, wherein the cationic lipid comprises 1,2-dimyristoyl-sn-glycero-3-ethylphosphatidylcholine (EDMPC⁺) or a pharmaceutically acceptable salt thereof.
(31) A dispersion formulation comprising a particle comprising a solid complex of a molecule of interest (preferably a water-soluble molecule) and a lipid mixture (comprising one or more lipidic surfactants) comprising 1,2-dimyristoyl-sn-glycero-3-ethylphosphatidylcholine (EDMPC⁺) or a pharmaceutically acceptable salt thereof, and an oily base, wherein the particle is dispersed in the oily base.
(32) The dispersion formulation according to (31) above, wherein the lipid mixture consists of 1,2-dimyristoyl-sn-glycero-3-ethylphosphatidylcholine (EDMPC⁺) or a pharmaceutically acceptable salt thereof (preferably a chloride salt).
(33) The dispersion formulation according to (31) or (32) above, wherein the pharmaceutically acceptable salt is a chloride salt of EDMPC⁺ (EDMPC⁺ Cl⁻).
(34) The dispersion formulation according to any one of (31) to (33) above, wherein the lipid mixture further comprises an anionic lipid.
(35) The dispersion formulation according to (34) above, wherein the anionic lipid comprises any one or more selected from the group consisting of oleic acid (OA), 1,2-dioleoyl-sn-glycero-3-phosphatidic acid (DOPA), 1,2-dioleoyl-sn-glycero-3-phosphoglycerol (DOPG), cardiolipin (CL), 1,2-di-(9Z-octadecenoyl)-sn-glycero-3-phospho-L-serine (DOPS), and phosphatidylinositol (PI).
(41) A composition comprising a complex of a molecule of interest (e.g., a water-soluble molecule) and a lipid mixture comprising 1,2-dimyristoyl-sn-glycero-3-ethylphosphatidylcholine (EDMPC⁺) or a pharmaceutically acceptable salt thereof and a lipid vesicle (e.g., extracellular vesicle).
(42) The composition according to (41) above, wherein the pharmaceutically acceptable salt is a chloride salt of EDMPC⁺ (EDMPC⁺ Cl⁻).
(43) The composition according to (41) or (42) above, wherein the lipid mixture consists of 1,2-dimyristoyl-sn-glycero-3-ethylphosphatidylcholine (EDMPC⁺) or a pharmaceutically acceptable salt thereof (preferably a chloride salt).
(44) The composition according to any one of (41) to (43) above, wherein the lipid mixture further comprises an anionic lipid.
(45) The composition according to (44) above, wherein the anionic lipid comprises any one or more selected from the group consisting of oleic acid (OA), 1,2-dioleoyl-sn-glycero-3-phosphatidic acid (DOPA), 1,2-dioleoyl-sn-glycero-3-phosphoglycerol (DOPG), cardiolipin (CL), 1,2-di-(9Z-octadecenoyl)-sn-glycero-3-phospho-L-serine (DOPS), and phosphatidylinositol (PI).
(46) The composition according to any one of (41) to (45) above, wherein the lipid mixture and the lipid vesicle (e.g., extracellular vesicle) form a vesicle, and the molecule of interest is encapsulated in the vesicle.
(47) The composition according to any one of (41) to (46) above, wherein the molecule of interest is a polypeptide.
(48) The composition according to any one of (41) to (46) above, wherein the molecule of interest is a nucleic acid.
(49) The composition according to any one of (41) to (46) above, wherein the molecule of interest is an antibody or an antigen-binding fragment thereof.
(50) The composition according to any one of (41) to (49) above, for delivering a molecule of interest to a cell.
(51) The composition according to any one of (41) to (50) above, wherein the complex is obtained by a method comprising: obtaining a complex (first complex) of a molecule of interest and a lipid mixture; and contacting the obtained complex with a lipid vesicle (e.g., extracellular vesicle) to obtain a composite particle (second complex) of the complex and the lipid vesicle (e.g., extracellular vesicle), wherein the molecule of interest is encapsulated in the lipid vesicle (e.g., extracellular vesicle).
(61) The dispersion formulation according to (3) to (10), (21), and (22) above, wherein the cationic lipid comprises 1,2-dimyristoyl-sn-glycero-3-ethylphosphatidylcholine (EDMPC⁺) or a pharmaceutically acceptable salt thereof.
(71) The composition according to any one of (1) to (9) above, for use in delivering a molecule of interest to the brain.
(72) The composition according to any one of (1) to (9) above, for use in delivering a molecule of interest into brain cells.
(73) The composition according to any one of (1) to (9) above, for use in delivering a molecule of interest to muscle.
(74) The composition according to any one of (1) to (9) above, for use in delivering a molecule of interest to the myocardium.
(75) The composition according to any one of (1) to (9) above, for use in delivering a molecule of interest to the pancreas.
(76) The composition according to any one of (1) to (9) above, for use in delivering a molecule of interest to the ovary.
(77) The composition according to any one of (1) to (9) above, for use in delivering a molecule of interest to the spleen.
(78) The composition according to any one of (1) to (9) above, for use in delivering a molecule of interest to the kidney.
(81) The composition according to any one of (41) to (49) above, for use in delivering a molecule of interest to the brain.
(82) The composition according to any one of (41) to (49) above, for use in delivering a molecule of interest to the lung.
(83) The composition according to any one of (41) to (49) above, for use in delivering a molecule of interest to muscle.
(84) The composition according to any one of (41) to (49) above, for use in delivering a molecule of interest to the heart.
(85) The composition according to any one of (41) to (49) above, for use in delivering a molecule of interest to the spleen.
(86) The composition according to any one of (41) to (49) above, for use in delivering a molecule of interest to the kidney.
(91) The composition according to any one of (1) to (9) above, for use in treating a cerebral neurological disease in a subject {wherein the molecule of interest provides a therapeutic benefit against the cerebral neurological disease}.
(93) The composition according to any one of (1) to (9) above, for use in treating a muscle disease in a subject {wherein the molecule of interest provides a therapeutic benefit against the muscle disease}.
(94) The composition according to any one of (1) to (9) above, for use in treating a cardiac disease in a subject {wherein the molecule of interest provides a therapeutic benefit against the cardiac disease}.
(95) The composition according to any one of (1) to (9) above, for use in treating a pancreatic disease in a subject {wherein the molecule of interest provides a therapeutic benefit against the pancreatic disease}.
(96) The composition according to any one of (1) to (9) above, for use in treating an ovarian disease in a subject {wherein the molecule of interest provides a therapeutic benefit against the ovarian disease}.
(97) The composition according to any one of (1) to (9) above, for use in treating a splenic disease in a subject {wherein the molecule of interest provides a therapeutic benefit against the splenic disease}.
(98) The composition according to any one of (1) to (9) above, for use in activating antigen-specific immunity in a subject {wherein the molecule of interest may be an antigen or a nucleic acid (e.g., DNA or RNA) encoding an antigen}.
(99) The composition according to any one of (1) to (9) above, for use in treating a renal disease in a subject {wherein the molecule of interest provides a therapeutic benefit against the renal disease}.
(111) The composition according to any one of (41) to (49) above, for use in treating a cerebral neurological disease in a subject {wherein the molecule of interest provides a therapeutic benefit against the cerebral neurological disease}.
(112) The composition according to any one of (41) to (49) above, for use in treating a pulmonary disease in a subject {wherein the molecule of interest provides a therapeutic benefit against the pulmonary disease}.
(113) The composition according to any one of (41) to (49) above, for use in treating a muscle disease in a subject {wherein the molecule of interest provides a therapeutic benefit against the muscle disease}.
(114) The composition according to any one of (41) to (49) above, for use in treating a cardiac disease in a subject {wherein the molecule of interest provides a therapeutic benefit against the cardiac disease}.
(115) The composition according to any one of (41) to (49) above, for use in treating a pancreatic disease in a subject {wherein the molecule of interest provides a therapeutic benefit against the pancreatic disease}.
(116) The composition according to any one of (41) to (49) above, for use in activating antigen-specific immunity in a subject {wherein the molecule of interest may be an antigen or a nucleic acid (e.g., DNA or RNA) encoding an antigen}.
(117) The composition according to any one of (41) to (49) above, for use in delivering a molecule of interest to the kidney {wherein the molecule of interest provides a therapeutic benefit against a renal disease}.
(120) A dispersion solution comprising (i) a complex of a molecule of interest and a lipid and (ii) an aqueous base or an oily base, wherein the complex is dispersed in the base. Here, the molecule of interest may preferably be an antibody. The lipid may be an ionic lipid and may preferably comprise a cationic lipid. In certain embodiments, when the dispersion solution comprises an oily base, the dispersion solution is not an ophthalmic composition for eye drop or intravitreal administration.
(121) An aqueous dispersion solution comprising a complex of a lipid and an antibody and an aqueous base, wherein the complex is dispersed in the aqueous base.
(122) The aqueous dispersion solution according to (121) above, wherein the lipid comprises or consists of a cationic lipid.
(123) The aqueous dispersion solution according to (121) or (122) above, wherein the lipid comprises or consists of 1,2-dimyristoyl-sn-glycero-3-ethylphosphatidylcholine (EDMPC⁺) or a pharmaceutically acceptable salt thereof (preferably a chloride salt).
(124) The aqueous dispersion solution according to any one of (121) to (123) above, wherein the lipid further comprises an anionic lipid.
(125) The aqueous dispersion solution according to (124) above, wherein the anionic lipid comprises linoleic acid.
(126) The aqueous dispersion solution according to any one of (121) to (125) above, wherein the aqueous base is physiological saline.
(127) The aqueous dispersion solution according to any one of (121) to (125) above, wherein the aqueous base is a medium suitable for introducing a reagent into cells (e.g., a serum-free medium).
(131) An oily dispersion solution comprising a solid complex of a lipid and an antibody and an oily base, wherein the complex is dispersed in the oily base {excluding ophthalmic formulations}.
(132) The oily dispersion solution according to (131) above, wherein the lipid comprises or consists of a cationic lipid.
(133) The oily dispersion solution according to (131) or (132) above, wherein the lipid comprises or consists of 1,2-dimyristoyl-sn-glycero-3-ethylphosphatidylcholine (EDMPC⁺) or a pharmaceutically acceptable salt thereof (preferably a chloride salt).
(134) The oily dispersion solution according to any one of (131) to (133) above, wherein the lipid further comprises an anionic lipid.
(135) The oily dispersion solution according to any one of (131) to (134) above, wherein the anionic lipid comprises linoleic acid.
(136) The oily dispersion solution according to any one of (131) to (135) above, wherein the oily base comprises or consists of isopropyl myristate.

### BRIEF DESCRIPTION OF DRAWINGS

[FIG. 1A] FIG. 1A shows the particle size distribution of extracellular vesicles, the particle size distribution of a lipid-anti-IL-2 antibody complex (first complex), and the particle size distribution of a complex (second complex) of extracellular vesicles and the first complex. The extracellular vesicles are the smallest, followed by the first complex at an intermediate size, and the second complex is the largest.
[FIG. 1B] FIG. 1B shows the results of a test in which a lipid-peptide molecule complex (first complex) was formed, a complex (second complex) of extracellular vesicles and the first complex was further formed, and the ratio of peptide-loaded extracellular vesicles to extracellular vesicles was examined.
[FIG. 1C] FIG. 1C shows the results of a test in which a lipid-nucleic acid molecule complex (first complex) was formed, a complex (second complex) of extracellular vesicles and the first complex was further formed, and the ratio of nucleic acid-loaded extracellular vesicles to extracellular vesicles was examined.
[FIG. 1D] FIG. 1D shows the results of a test in which a lipid-RNP molecule complex (first complex) was formed, a complex (second complex) of extracellular vesicles and the first complex was further formed, and the ratio of RNP-loaded extracellular vesicles to extracellular vesicles was examined. RNP means an RNA-protein complex (RNP), and in this experiment, a Cas9-gRNA complex was used as the RNP.
[FIG. 1E] FIG. 1E shows that, by forming a lipid-antibody molecule complex (first complex), further forming a complex (second complex) of extracellular vesicles and the first complex, and subjecting the obtained solution to protease digestion treatment, the antibody is encapsulated in the vesicle in the second complex.
[FIG. 1F] FIG. 1F shows that, by forming a lipid-microRNA molecule complex (first complex), further forming a complex (second complex) of extracellular vesicles and the first complex, and subjecting the obtained solution to RNA digestion enzyme treatment, the microRNA is encapsulated in the vesicle in the second complex.
[FIG. 2] FIG. 2 shows the results of immunostaining of a lipid-molecule of interest complex (first complex) and a complex (second complex) of extracellular vesicles and the first complex.
[FIG. 3A] FIG. 3A shows the results of immunostaining of cells contacted with a lipid-molecule of interest complex (first complex) and a complex (second complex) of extracellular vesicles and the first complex, respectively.
[FIG. 3B] FIG. 3B shows the results of immunostaining of cells contacted with a complex (first complex) of a single type of lipid and a molecule of interest and a complex (second complex) of extracellular vesicles and the first complex, respectively.
[FIG. 4A] FIG. 4A shows the proportion of cells in which the antibody was delivered intracellularly among cells contacted with a first complex or a second complex containing an antibody, respectively.
[FIG. 4B] FIG. 4B shows fluorescence microscope images of cells in which the antibody was delivered intracellularly among cells contacted with a first complex or a second complex containing an antibody, respectively.
[FIG. 4C] FIG. 4C shows the delivery of peptide into cells contacted with a first complex or a second complex containing a peptide, respectively.
[FIG. 5] FIG. 5 shows the amount of siRNA introduced into cells contacted with a first complex or a second complex containing siRNA, respectively.
[FIG. 6A] FIG. 6A shows the invasion rate of cells to the lower part of the chamber after cell delivery of an anti-matrix metalloproteinase-7 antibody (anti-MMP-7 antibody) or various complexes containing the antibody.
[FIG. 6B] FIG. 6B shows staining images of cells that invaded the lower part of the chamber after cell delivery of an anti-matrix metalloproteinase-7 antibody (anti-MMP-7 antibody) or various complexes containing the antibody.
[FIG. 6C] FIG. 6C shows the effect of treatment with a first complex or a second complex containing an anti-MMP-7 antibody on cell migration ability. Specifically, FIG. 6C shows the invasion rate of cells to the lower part of the chamber after cell delivery of an anti-matrix metalloproteinase-7 antibody (anti-MMP-7 antibody) or various complexes containing the antibody.
[FIG. 6D] FIG. 6D shows the effect of treatment with a first complex or a second complex containing an anti-MMP-7 antibody on cell proliferation.
[FIG. 7] FIG. 7 shows staining images of cells into which antisense oligonucleotides (ASO) were introduced by the first complex and the second complex.
[FIG. 8] FIG. 8 shows the particle size distributions of the prepared exosomes, first complex, and second complex measured by dynamic light scattering (DLS).

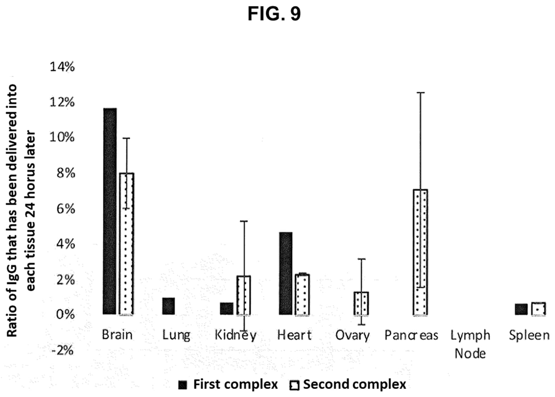

[FIG. 9] FIG. 9 shows the tissue distribution of the first complex and the second complex after tail vein injection administration.
[FIG. 10A] FIG. 10A shows the distribution of the first complex administered by tail vein injection in brain tissue. The dashed-line box indicates a region in the vicinity of blood vessels (CD31-positive), and the solid-line box indicates a region distant from blood vessels. IgG was labeled with Cy5, and the presence of antibodies was confirmed within the dashed-line box and the solid-line box.
[FIG. 10B] FIG. 10B shows the distribution of the second complex administered by tail vein injection in brain tissue. The solid-line box indicates a region distant from blood vessels. IgG was labeled with Cy5, and the presence of antibodies was confirmed within the dashed-line box and the solid-line box.
[FIG. 11A] FIG. 11A shows the distribution of antibody molecules in the brain 48 hours after tail vein injection to mice of a solution obtained by forming a lipid-antibody molecule complex (first complex) and further forming a complex (second complex) of extracellular vesicles and the first complex.
[FIG. 11B] FIG. 11B is an enlarged view of a portion of FIG. 11A. In the right column of FIG. 11B, the localization of antibodies is indicated by arrows based on Cy5 fluorescence.
[FIG. 11C] FIG. 11C is an enlarged view of a portion of FIG. 11A. The cerebral cortex is shown, and the uptake of antibodies by microglia cells is indicated by arrows.

### DESCRIPTION OF EMBODIMENTS

Embodiments according to the present invention will be described with reference to the drawings. Note that the present invention is not limited by the following embodiments and drawings. In the following embodiments, expressions such as "having," "comprising," or "containing" mean that the composition may include unspecified third components, and also encompass the meaning of "consisting of," which means essentially not including a third component.

In the present specification, a "lipid vesicle" is a vesicle having a lipid membrane structure. A lipid vesicle typically has a lipid bilayer. A lipid vesicle may comprise an artificial lipid, but may also be one that does not comprise an artificial lipid, may consist of natural lipids with respect to lipids, may be, for example, one that is naturally produced, and preferably may be one produced by cells (e.g., extracellular vesicles). The size of the lipid vesicle is not particularly limited, but may have, for example, a sub-micrometer diameter. The lipid vesicle may be a particle having a diameter of, for example, about 30 nm to about 150 nm. The lipid vesicle may be, for example, a liposome.

In the present specification, an "extracellular vesicle" is a particle having a membrane structure secreted by a cell. Extracellular vesicles are considered to mediate intercellular communication. Extracellular vesicles are broadly classified into three types: exosomes, microvesicles, and apoptotic bodies. Exosomes are particles having a diameter of about 30 nm to about 150 nm and are considered to be secreted from multivesicular bodies (MVBs) formed inside endosomes. Exosomes are considered to have the ability to carry genetic material (such as mRNA and miRNA), proteins, and lipids from cells, thereby being able to influence the functions and phenotypes of recipient cells. Apoptotic bodies are extracellular vesicles having a diameter of about 500 nm to about 2000 nm and released when cells are degraded during the process of programmed cell death (apoptosis). Apoptotic bodies contain the genetic material, organelles, and parts of the cell membrane of cells, and are considered to be taken up and cleared by adjacent cells and cells of the immune system. Microvesicles are particles having a diameter of about 100 nm to about 1000 nm produced by a mechanism different from that of exosomes and apoptotic bodies. Generated microvesicles contain parts of the cell membrane, cytoplasmic components (proteins, lipoproteins, genetic material, etc.). Microvesicles are considered to play important roles in intercellular communication and influence the functions and phenotypes of adjacent cells and distant cells through the transport of genetic material and proteins. It is considered that by introducing substances into microvesicles or exosomes, substances can be delivered to cells.

In the present specification, "cation" means a molecule having a positive charge under pH 7 conditions. In the present specification, "cationic" means a property of having a positive charge under pH 7 conditions. In the present specification, "anion" means a molecule having a negative charge under pH 7 conditions. In the present specification, "anionic" means a property of having a negative charge under pH 7 conditions.

In the present specification, a "surfactant" is a molecule having a relatively hydrophilic moiety and a relatively hydrophobic moiety in the molecule. Surfactants are broadly classified into ionic surfactants and nonionic surfactants. Ionic surfactants include cationic surfactants and anionic surfactants. Typically, an ionic surfactant may have either one of a cation and an anion and a hydrophobic moiety. A cationic surfactant may comprise a cationic moiety and a fatty chain (e.g., a long-chain fatty chain), e.g., alkyl, alkenyl, or alkynyl. An anionic surfactant may comprise an anionic moiety and a fatty acid such as a long-chain fatty acid, an unsaturated fatty acid (e.g., monounsaturated or diunsaturated), or a long-chain unsaturated fatty acid (e.g., monounsaturated or diunsaturated). "Long-chain" is a term given to compounds having 14 to 22 carbon atoms. The fatty chain may be a fatty chain having 8 to 22 carbon atoms, that is, a C₈ to C₂₂ fatty chain such as C₈ to C₂₂ alkyl, C₈ to C₂₂ alkenyl, or C₈ to C₂₂ alkynyl. The fatty chain may also be a long-chain fatty chain. Saturated means that the fatty chain has no double bonds or triple bonds, and unsaturated means that the fatty chain has at least one double bond or triple bond. Fatty chains having a double bond include cis-type and trans-type, and from the viewpoint of biocompatibility, cis-type is preferred. Among ionic surfactants, molecules in which the hydrophobic moiety is a lipid (fatty chain) are referred to as ionic lipids. In the present specification, a lipid mixture refers to a composition comprising one or two or more lipids.

As the surfactant (e.g., nonionic lipid or ionic lipid), a lipophilic nonionic lipid or ionic surfactant (e.g., ionic lipid, particularly cationic lipid and anionic lipid) having an HLB (Hydrophile-Lipophile Balance) value of 10 or less is preferred because the complex can be easily dispersed in the oil phase. The HLB of the nonionic lipid or ionic surfactant (e.g., ionic lipid, preferably cationic lipid and anionic lipid) is preferably 8 or less, more preferably 5 or less, and particularly preferably 3 or less. The HLB of the nonionic lipid or ionic surfactant (e.g., ionic lipid, preferably cationic lipid and anionic lipid) may be, for example, 1 to 3, 3 to 5, or 5 to 10. In certain embodiments, the HLB of the nonionic lipid or ionic surfactant (e.g., ionic lipid, preferably cationic lipid and anionic lipid) may be 5 to 10. In certain embodiments, the HLB of the nonionic lipid or cationic surfactant (e.g., cationic lipid) may be 1 to 10, for example, 1 to 3, 3 to 5, or 5 to 10. In certain embodiments, the HLB of the nonionic lipid or cationic surfactant (e.g., cationic lipid) may be 5 to 10. In certain embodiments, the HLB of the nonionic lipid or anionic surfactant (e.g., anionic lipid) may be 1 to 10, for example, 1 to 3, 3 to 5, or 5 to 10. In certain embodiments, the HLB of the nonionic lipid or anionic surfactant (e.g., anionic lipid) may be 5 to 10.

A lipid is a molecule of biological origin that is soluble in nonpolar solvents. Lipids can be classified into glycerolipids, glycerophospholipids, sphingolipids, sterol lipids, prenol lipids, glycolipids, and polyketides. Glycerolipids are the general term for molecules in which fatty acids are ester-bonded to the hydroxyl groups of glycerol. Glycerol has three hydroxyl groups, and up to three fatty acids can be linked by ester bonds. In glycerophospholipids, two fatty acids are ester-bonded to two hydroxyl groups of glycerol, and a phosphate group is linked to one hydroxyl group. Sphingolipids may be ceramides, sphingophospholipids, or sphingoglycolipids. Sterol lipids are a subgroup of terpenoids. Sterol lipids may be sterols (e.g., cholesterol). Prenol lipids are terpenoids other than sterol lipids. Nonionic lipids comprise a hydrophilic moiety and a hydrophobic moiety (particularly a lipid moiety).

Cationic lipids are described in U.S. Patent Application Publication Nos. 2006/0083780 and 2006/0240554, U.S. Patent Nos. 5,208,036, 5,264,618, 5,279,833, 5,283,185, 5,753,613, and 5,785,992, and PCT Publication No. WO96/10390, the disclosures of which are incorporated herein by reference in their entirety for all purposes. Examples of cationic lipids include 1,2-dilinoleyloxy-N,N-dimethylaminopropane (DLinDMA), 1,2-dilinolenyloxy-N,N-dimethylaminopropane (DLenDMA), 2,2-dilinoleyl-4-(2-dimethylaminoethyl)-[1,3]-dioxolane (DLin-K-C2-DMA; "XTC2"), 2,2-dilinoleyl-4-(3-dimethylaminopropyl)-[1,3]-dioxolane (DLin-K-C3-DMA), 2,2-dilinoleyl-4-(4-dimethylaminobutyl)-[1,3]-dioxolane (DLin-K-C4-DMA), 2,2-dilinoleyl-5-dimethylaminomethyl-[1,3]-dioxane (DLin-K6-DMA), 2,2-dilinoleyl-4-N-methylpiperazino-[1,3]-dioxolane (DLin-K-MPZ), 2,2-dilinoleyl-4-dimethylaminomethyl-[1,3]-dioxolane (DLin-K-DMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinolelylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N-(N',N'-dimethylaminoethane)-carbamoyl)cholesterol (DC-Chol), N-(1,2-dimyristyloxyprop-3-yl)-N,N-dimethyl-N-hydroxyethylammonium bromide (DMRIE), 2,3-dioleyloxy-N-[2(spermine-carboxamido)ethyl]-N,N-dimethyl-1-propanaminium trifluoroacetate (DOSPA), dioctadecylamidoglycylspermine (DOGS), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethyl-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), or mixtures thereof. In certain preferred embodiments, the cationic lipid is DLinDMA, DLin-K-C2-DMA ("XTC2"), or a mixture thereof. Preferred cationic lipids include 1,2-dimyristoyl-sn-glycero-3-ethylphosphatidylcholine (EDMPC⁺). Cationic lipids may have, for example, a phosphate ester moiety (e.g., non-charged) and a quaternary ammonium group.

Preferred anionic lipids include those represented by General Formula (1):

R-COO⁻X⁺ ... (1).

In General Formula (1), R represents a substituted or unsubstituted alkyl group, or a substituted or unsubstituted alkenyl group, and at least one ethylene group constituting the alkenyl group may be substituted with a vinylene group.

The alkyl group in R may be any of linear, branched, and cyclic. The preferred number of carbon atoms of the alkyl group in R is 8 to 22, more preferably 12 to 22, and still more preferably 14 to 22. For example, examples of the alkyl group in R include linear alkyl groups such as dodecyl, tridecyl, tetradecyl, pentadecyl, hexadecyl, heptadecyl, octadecyl, nonadecyl, icosyl, henicosyl, and docosyl groups, branched alkyl groups thereof, and cyclic alkyl groups thereof. Examples of substituents that can substitute on the alkyl group include amino groups, benzyl groups, and halogen atoms (e.g., fluorine atoms). These substituents may be further substituted with substituents. When the alkyl group is substituted with a substituent, the total of the number of carbon atoms of the alkyl group and the number of carbon atoms of the substituent is preferably 8 to 22, more preferably 12 to 22, and still more preferably 14 to 22.

The alkenyl group in R may be either linear or branched. The preferred number of carbon atoms of the alkenyl group in R is 8 to 22, more preferably 12 to 22, and still more preferably 14 to 22. Examples of the alkenyl group in R include linear alkenyl groups such as dodecenyl, tridecenyl, tetradecenyl, pentadecenyl, hexadecenyl, heptadecenyl, octadecenyl, nonadecenyl, icosenyl, henicosenyl, and docosenyl groups, and branched alkenyl groups thereof. Examples of substituents that can substitute on the alkenyl group include amino groups, benzyl groups, and halogen atoms (e.g., fluorine atoms). These substituents may be further substituted with substituents. When the alkenyl group is substituted with a substituent, the total of the number of carbon atoms of the alkenyl group and the number of carbon atoms of the substituent is preferably 8 to 22, more preferably 12 to 22, and still more preferably 14 to 22.

The alkenyl group in R may have at least one of its ethylene groups substituted with a vinylene group to form a polyene structure. The number of double bonds in the polyene structure is preferably 2 to 6, more preferably 2 to 4. The positions of the double bonds are not particularly limited, but it is preferable that double bonds are disposed with at least two single bonds between them. Among these, R is preferably a group having an unsaturated bond, more preferably a substituted or unsubstituted alkenyl group or a group having a substituted or unsubstituted polyene structure.

The alkyl group, alkenyl group, and group having a polyene structure in R may be substituted with substituents, but even in that case, R preferably consists only of carbon atoms and hydrogen atoms. That is, when the alkyl group, alkenyl group, and polyene structure are substituted with substituents, the substituents also preferably consist only of carbon atoms and hydrogen atoms.

For R-COO⁻, for example, a carboxylate ion in which a hydrogen ion has dissociated from the carboxy group of a fatty acid, or a derivative thereof, can be used. The fatty acid that produces a carboxylate ion may be a saturated fatty acid or an unsaturated fatty acid, but is preferably an unsaturated fatty acid, more preferably a cis-type unsaturated fatty acid, and still more preferably a cis-type long-chain unsaturated fatty acid. Examples of saturated fatty acids include myristic acid (C14:0), palmitic acid (C16:0), stearic acid (C18:0), and lauric acid (C12:0), and examples of unsaturated fatty acids include oleic acid (C18:1), linoleic acid (C18:2), alpha-linolenic acid (C18:3), gamma-linolenic acid (C18:3), arachidonic acid (C20:4), eicosapentaenoic acid (C20:5), docosahexaenoic acid (C22:6), and erucic acid (C22: 1). Here, the numerical values in parentheses represent the number of carbon atoms and the number of double bonds of each fatty acid. For example, (C18:2) of linoleic acid indicates that the number of carbon atoms is 18 and the number of double bonds is 2. In General Formula (1), X⁺ represents a phospholipid having a cationic group. Here, "phospholipid" means a lipid comprising a phosphate ester structure, and "cationic group" means a substituent bearing a positive charge. X+ is preferably a glycerophospholipid having a cationic group, and more preferably a derivative of phosphatidylcholine. Here, a derivative of phosphatidylcholine refers to a compound having a glycerol backbone, substituted or unsubstituted alkanoyl groups bonded to the 1-position and 2-position of the glycerol backbone, respectively, a phosphate ester group (-P(=O)(OH)O⁻) bonded to the 3-position of the glycerol backbone, and a choline residue bonded to this phosphate ester group. In this phosphatidylcholine derivative, at least one ethylene group of the alkanoyl group may be replaced with a vinylene group, and the hydrogen atom of the hydroxyl group of the phosphate ester group may be substituted with a substituted or unsubstituted alkyl group.

X⁺ preferably has a phospholipid having a structure represented by the following General Formula (2), and more preferably is a glycerophospholipid having a structure represented by General Formula (2).

CH(-CH₂-O-C(=O)-R¹⁰)(-O-C(=O)-R¹¹)-CH₂-O-P(OR¹)(OR²)(=O) ... (2)

In General Formula (2), R¹ represents an alkyl group substituted with a cationic group, and R² represents a hydrogen atom or a substituted or unsubstituted alkyl group. The phospholipid having a structure represented by General Formula (2) has an overall positive charge and preferably has no negatively charged moiety.

The alkyl group in R¹ may be any of linear, branched, and cyclic. The preferred number of carbon atoms of the alkyl group in R¹ is 1 to 20, more preferably 1 to 10, and still more preferably 1 to 6. For example, examples of the alkyl group in R¹ include methyl, ethyl, n-propyl, and isopropyl groups, and the alkyl group in R¹ is preferably an ethyl group.

The cationic group in R¹ is preferably an ammonium group represented by General Formula (3):

-NR⁴R⁵R⁶⁺ ... (3)

In General Formula (3), R⁴, R⁵, and R⁶ each independently represent a hydrogen atom or a substituted or unsubstituted alkyl group, and * represents the bonding position to the alkyl group. R⁴, R⁵, and R⁶ may be the same as or different from each other. The number of substituted or unsubstituted alkyl groups among R⁴, R⁵, and R⁶ is not particularly limited, and all of R⁴, R⁵, and R⁶ may be hydrogen atoms or substituted or unsubstituted alkyl groups, or one or two of them may be hydrogen atoms and the remainder may be substituted or unsubstituted alkyl groups.

The alkyl group in R⁴, R⁵, and R⁶ may be any of linear, branched, and cyclic. The preferred number of carbon atoms of the alkyl group in R⁴, R⁵, and R⁶ is 1 to 20, more preferably 1 to 10, and still more preferably 1 to 6. For example, examples of the alkyl group in R⁴, R⁵, and R⁶ include methyl, ethyl, n-propyl, and isopropyl groups, and the alkyl group in R⁴, R⁵, and R⁶ is preferably a methyl group. Examples of substituents that can substitute on the alkyl group include benzyl groups and halogen atoms (e.g., fluorine atoms). These substituents may be further substituted with substituents. In certain preferred embodiments, R⁴, R⁵, and R⁶ are methyl groups.

The cationic group in R¹ is preferably a quaternary ammonium group, and more preferably a quaternary ammonium group represented by General Formula (5) (an ammonium group in which all of R⁴ are substituted or unsubstituted alkyl groups). Furthermore, the position of substitution of the cationic group on the alkyl group is not particularly limited, but it is preferable that a hydrogen atom bonded to a carbon atom at the terminal of the alkyl group is substituted with a cationic group.

In General Formula (2), R² represents a hydrogen atom or a substituted or unsubstituted alkyl group. R² is preferably a substituted or unsubstituted alkyl group. The alkyl group in R² is preferably an ethyl group. The alkyl group in R² may be any of linear, branched, and cyclic. The preferred number of carbon atoms of the alkyl group in R² is 1 to 20, more preferably 1 to 10, and still more preferably 1 to 6. For example, examples of the alkyl group in R² include methyl, ethyl, n-propyl, and isopropyl groups, and the alkyl group in R² is preferably an ethyl group. Examples of substituents that can substitute on the alkyl group include benzyl groups and halogen atoms (e.g., fluorine atoms). These substituents may be further substituted with substituents.

In General Formula (2), R¹⁰ and R¹¹ are each independently a fatty chain having 8 to 22 carbon atoms, and are preferably long-chain fatty chains, which may be long-chain saturated fatty chains or long-chain unsaturated fatty chains. In certain embodiments, R¹⁰ and R¹¹ are each independently a long-chain saturated fatty chain having 12 to 22 carbon atoms. In certain embodiments, R¹⁰ and R¹¹ may be long-chain saturated fatty chains having 12 to 14 (e.g., 13) carbon atoms. In certain embodiments, the glycerophospholipid represented by General Formula (2) may be EDMPC⁺.

The number of substitutions by alkylcarbonyloxy groups on the alkyl group is preferably 2 to 8, more preferably 2 to 4, and most preferably 2. The substitution position of the alkylcarbonyloxy group on the alkyl group is not particularly limited; however, when the alkyl group as the main chain of R³ is an n-propyl group, those in which the hydrogen atom bonded to the terminal carbon atom and the hydrogen atom bonded to the adjacent carbon atom are substituted with alkylcarbonyloxy groups correspond to glycerophospholipids, which are particularly preferred phospholipids.

For the description, preferred ranges, and specific examples of the alkyl group in the alkylcarbonyloxy group, reference can be made to the description of the alkyl group in R above.

Preferred examples of compounds having a structure represented by General Formula (1) are given below. However, the ionic surfactant according to the present embodiment should not be interpreted as limited by these specific examples.
For example, the carboxylate ion may be a long-chain fatty acid, preferably a long-chain unsaturated fatty acid, such as linoleic acid or oleic acid, or a long-chain saturated fatty acid, such as stearic acid. In the glycerophospholipid represented by General Formula (2), R¹ is -NR⁴R⁵R⁶, wherein R⁴, R⁵, and R⁶ are each independently methyl or ethyl, preferably all are methyl, and R¹⁰ and R¹¹ are each independently a long-chain fatty chain, preferably each independently a long-chain fatty chain having 12 to 22 carbon atoms, and more preferably a long-chain fatty chain having 12 to 14 carbon atoms. As a more specific example, for instance, the carboxylate ion is a long-chain unsaturated fatty acid, such as linoleic acid or oleic acid, and in the glycerophospholipid represented by General Formula (2), R¹ is -NR⁴R⁵R⁶, wherein R⁴, R⁵, and R⁶ are each independently methyl or ethyl, preferably all are methyl, and R¹⁰ and R¹¹ are each independently a long-chain fatty chain having 12 to 22 carbon atoms, and more preferably a long-chain fatty chain having 12 to 14 carbon atoms. As an even more specific example, for instance, the carboxylate ion is a long-chain unsaturated fatty acid, such as linoleic acid or oleic acid, and in the glycerophospholipid represented by General Formula (2), R¹ is -NR⁴R⁵R⁶, wherein R⁴, R⁵, and R⁶ are methyl, and R¹⁰ and R¹¹ are each independently a long-chain fatty chain having 12 to 22 carbon atoms, and more preferably a long-chain fatty chain having 12 to 14 carbon atoms. As an even more specific example, in the following formula, R-COO⁻has the same meaning as R-COO⁻ in General Formula (1), and specific examples thereof include carboxylate ions of linoleic acid, oleic acid, acetic acid, or stearic acid.

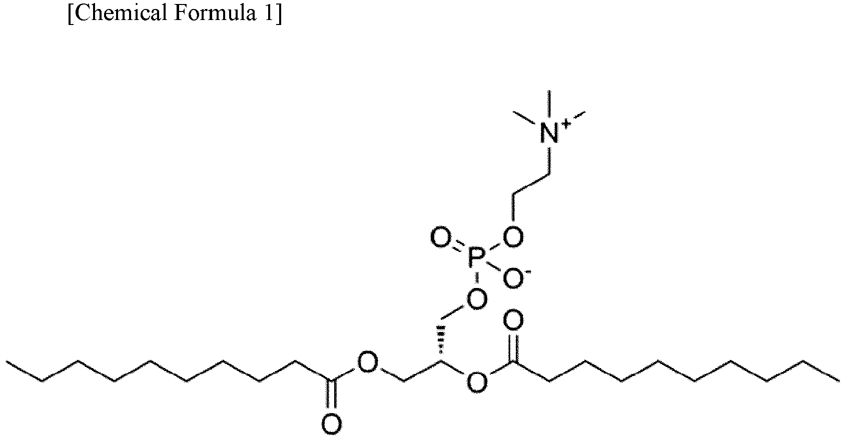

The above shows the manner in which a carboxylate ion and a preferred cationic surfactant form an ionic bond. A mixture of these cationic lipids and anionic lipids may be a liquid (particularly an ionic liquid) at room temperature when mixed at a 1:1 charge ratio.

Examples of anionic surfactants include phospholipids and fatty acids, including phosphatidylglycerol, cardiolipin, diacylphosphatidylserine, diacylphosphatidic acid, N-dodecanoylphosphatidylethanolamine, N-succinylphosphatidylethanolamine, N-glutarylphosphatidylethanolamine, lysylphosphatidylglycerol, palmitoyloleoylphosphatidylglycerol (POPG), and those in which other anionic modifying groups are bonded to neutral lipids.

Examples of phospholipids include phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine.

A fatty acid is a carbon chain having a monovalent carboxylic acid. A fatty acid is preferably cis-type rather than trans-type. Fatty acids are represented by the rational formula CH₃-R-CO₂H {where R is a carbon chain}, and are expressed based on the number of carbon atoms and the number of double bonds contained in the fatty acid. For example, capric acid, in which R is -(CH₂)₈-, is represented by 10:0; lauric acid, in which R is -(CH₂)₁₀-, is represented by 12:0; myristic acid, in which R is -(CH₂)₁₄-, is represented by 14:0; pentadecylic acid, in which R is -(CH₂)₁₅-, is represented by 15:0; palmitic acid, in which R is -(CH₂)₁₆-, is represented by 16:0; palmitoleic acid, in which R is -(CH₂)₅CH=CH(CH₂)₇-, is represented by 16:1; stearic acid, in which R is -(CH₂)₁₈-, is represented by 18:0; oleic acid, in which R is -(CH₂)₇CH=CH(CH₂)₇-, is represented by 18:1 (n-9); vaccenic acid, in which R is -(CH₂)₅CH=CH(CH₂)₉-, is represented by 18:1 (n-7); linoleic acid, in which R is -(CH₂)₃(CH₂CH=CH)₂(CH₂)₇-, is represented by 18:2 (n-6); (9,12,15)-linolenic acid, in which R is -(CH₂CH=CH)₃(CH₂)₇-, is represented by 18:3 (n-3); and (6,9,12)-linolenic acid, in which R is -(CH₂)₃(CH₂CH=CH)₃(CH₂)₄-, is represented by 18:3 (n-6).

In the present specification, "molecule of interest" refers to molecules such as polypeptides (proteins), nucleic acids, and small-molecule compounds, as well as complexes thereof, and preferably has physiological activity. The molecule of interest may be a fusion protein of a plurality of proteins, a complex of a plurality of proteins (e.g., an antibody), or a complex of a protein and a nucleic acid (e.g., an RNA-protein complex (RNP)). In preferred embodiments, the molecule of interest may be a water-soluble molecule. In the present specification, "water-soluble molecule" refers to a molecule having water solubility and capable of achieving the expected effect by the molecule dissolved in an effective amount. A water-soluble molecule may have sufficient water solubility to achieve a concentration of, for example, 100 µM or less, 50 µM or less, 40 µM or less, 30 µM or less, 20 µM or less, 10 µM or less, 5 µM or less, 4 µM or less, 3 µM or less, 2 µM or less, 1 µM or less, 500 nM or less, 400 nM or less, 300 nM or less, 200 nM or less, 100 nM or less, 50 nM or less, 40 nM or less, 30 nM or less, 20 nM or less, 10 nM or less, 9 nM or less, 8 nM or less, 7 nM or less, 6 nM or less, 5 nM or less, 4 nM or less, 3 nM or less, 2 nM or less, or 1 nM or less. A water-soluble molecule may have sufficient water solubility to achieve a concentration of, for example, 1 pM or more, 10 pM or more, 100 pM or more, or 1 nM or more.

### (Embodiment 1)

According to the present disclosure, there is provided a dispersion formulation comprising a particle comprising a solid complex of a molecule (preferably a water-soluble molecule) and a lipid mixture (comprising one or more lipid surfactants) comprising 1,2-dimyristoyl-sn-glycero-3-ethylphosphatidylcholine (EDMPC⁺) or a pharmaceutically acceptable salt thereof, and an oily base, wherein the particle is dispersed in the oily base. In this embodiment, the lipid mixture may or may not comprise an anionic lipid. When an anionic lipid is comprised, the anionic lipid preferably comprises any one or more selected from the group consisting of oleic acid (OA), 1,2-dioleoyl-sn-glycero-3-phosphatidic acid (DOPA), 1,2-dioleoyl-sn-glycero-3-phosphoglycerol (DOPG), cardiolipin (CL), 1,2-di-(9Z-octadecenoyl)-sn-glycero-3-phospho-L-serine (DOPS), and phosphatidylinositol (PI). As a pharmaceutically acceptable salt of EDMPC⁺, a salt that neutralizes the charge of EDMPC⁺ is preferably used, and may be, for example, a chloride salt of EDMPC⁺ (EDMPC⁺ Cl⁻). This solid complex can also be advantageously used as a first complex described below. This solid complex can also be advantageously produced by the method for producing the first complex described below.

### (Embodiment 2)

According to the present disclosure, a complex (first complex) comprising a lipid (lipid mixture) and a molecule of interest is provided. According to the present disclosure, a complex comprising a nonionic lipid and a molecule of interest is provided. According to the present disclosure, a complex comprising an ionic lipid and a molecule of interest is provided. In one embodiment, the ionic lipid comprises 30 mol% or more, 40 mol% or more, 50 mol% or more, 60 mol% or more, 70 mol% or more, 80 mol% or more, or 90 mol% or more of a cationic lipid. In one embodiment, the ionic lipid comprises 30 mol% or more, 40 mol% or more, 50 mol% or more, 60 mol% or more, 70 mol% or more, 80 mol% or more, or 90 mol% or more of an anionic lipid. In one embodiment, the ionic lipid comprises a cationic lipid and an anionic lipid. The cationic lipid/anionic lipid ratio (molar ratio) in the ionic lipid can be, for example, 0.1 or more, 0.2 or more, 0.3 or more, 0.4 or more, 0.5 or more, 0.6 or more, 0.7 or more, 0.8 or more, 0.9 or more, 1 or more, 1.5 or more, 2 or more, 3 or more, 4 or more, 5 or more, 6 or more, 7 or more, 8 or more, 9 or more, or 10 or more, and can be, for example, 0.1 to 10, 0.2 to 5, 0.25 to 4, 0.33 to 3, 0.4 to 2.5, 0.5 to 2, 0.67 to 1.5, 0.75 to 1.33, 0.8 to 1.25, 0.9 to 1.1, or about 1.0. According to the present disclosure, a complex (molecule of interest-lipid complex) comprising a liquid (liquid lipid composition) consisting of a cationic lipid and an anionic lipid (hereinafter sometimes referred to as an ionic surfactant or simply a surfactant) and a molecule of interest is provided. Nonionic lipids form micellar particles in water, and the molecule of interest is encapsulated in the micellar particles. Within the micellar particles, the nonionic lipids are considered to be oriented with the lipid moiety toward the core and the hydrophilic moiety toward the outside. Cationic lipids and anionic lipids form micellar particles in water, and the molecule of interest is encapsulated in the micellar particles. Within the micellar particles, the cationic lipid and the anionic lipid are considered to be oriented with the lipid toward the core and the ionic moiety toward the outside. Within the micellar particles, the molecule of interest interacts with the ionic moiety and/or the lipid moiety. The first complex is preferably used for introducing a molecule of interest into extracellular vesicles (e.g., exosomes).

### (Embodiment 3)

According to the present disclosure, a dispersion formulation comprising a complex comprising an antibody and a lipid (lipid mixture), and a base, wherein the complex is dispersed in the base, is provided. In one preferred embodiment, the complex is a solid complex and the base is an oily base. In another preferred embodiment, the base is an aqueous base. In this embodiment where the base is an aqueous base, the complex may or may not contain water. In one preferred embodiment, the lipid is an ionic lipid. In one embodiment, the ionic lipid comprises 30 mol% or more, 40 mol% or more, 50 mol% or more, 60 mol% or more, 70 mol% or more, 80 mol% or more, or 90 mol% or more of a cationic lipid. In one embodiment, the ionic lipid comprises 30 mol% or more, 40 mol% or more, 50 mol% or more, 60 mol% or more, 70 mol% or more, 80 mol% or more, or 90 mol% or more of an anionic lipid. In one embodiment, the ionic lipid comprises a cationic lipid and an anionic lipid. The cationic lipid/anionic lipid ratio (molar ratio) in the ionic lipid can be, for example, 0.1 or more, 0.2 or more, 0.3 or more, 0.4 or more, 0.5 or more, 0.6 or more, 0.7 or more, 0.8 or more, 0.9 or more, 1 or more, 1.5 or more, 2 or more, 3 or more, 4 or more, 5 or more, 6 or more, 7 or more, 8 or more, 9 or more, or 10 or more, and can be, for example, 0.1 to 10, 0.2 to 5, 0.25 to 4, 0.33 to 3, 0.4 to 2.5, 0.5 to 2, 0.67 to 1.5, 0.75 to 1.33, 0.8 to 1.25, 0.9 to 1.1, or about 1.0. The complex of this embodiment may form a further complex with exosomes (or may comprise extracellular vesicles such as exosomes), or may not form a further complex with exosomes (or may not comprise extracellular vesicles such as exosomes). In the complex of this embodiment, all components may be synthesized (artificially synthesized) and preferably isolated. In one preferred embodiment, the antibody may be a recombinant antibody produced from an antibody-producing cell, and the lipid may be a synthesized lipid. The antibody contained in the formulation retains its binding property to an antigen even after its administration.

In all embodiments (Embodiments 1 to 3 and other embodiments), in a preferred embodiment, the molecule of interest is a water-soluble molecule, the lipid is a surfactant (lipidic surfactant), and preferably the lipid comprises a cationic lipid. The molecule of interest can be, for example, a water-soluble protein (e.g., glycoprotein, enzyme, antibody, etc.), a nucleic acid, or a water-soluble low-molecular-weight compound.

The first complex may consist of only the molecule of interest and the surfactant, or may comprise other components such as a stabilizer. The stabilizer is, for example, a hydrophilic protein and polysaccharide, and one having a molecular weight of 10,000 or more is preferred. By being coated with the surfactant together with the molecule of interest, the stabilizer can improve the stability of the complex and prevent leakage of the molecule of interest to the outside of the complex in the composition.

Examples of the protein include bioactive substances such as cytokines (e.g., interleukin-1 (IL-1), IL-2, IL-3, IL-4, IL-5, IL-6, IL-7, IL-8, IL-9, IL-10, IL-11, IL-12, IL-13, IL-14, IL-15, α-interferon, β-interferon, γ-interferon, angiostatin, thrombospondin, endostatin, GM-CSF, G-CSF, M-CSF, and tumor necrosis factor). Examples of the protein also include agalsidase beta, laronidase, alteplase, pabunafusp alfa, urokinase, idursulfase, sebelipase alfa, monteplase, asphotase alfa, imiglucerase, velaglucerase, agalsidase alfa, alglucosidase alfa, avalglucosidase alfa, idursulfase, galsulfase, elosulfase alfa, rasburicase, dornase alfa, asphotase alfa, collagenase, sebelipase, chondriase, elapegademase, cerliponase alfa, glucarpidase, vestronidase alfa, olipudase alfa, blood coagulation factors (e.g., I, II, III, IV, V, VI, VII, VIII, IX, X, XI, and XII), insulin, somatropin, erythropoietin, and chemokines. Examples of the protein further include Cas9 protein, RNA polymerase, transcription factors, ribosomes, cyclin-dependent kinases, and growth factors (e.g., epidermal growth factor (EGF), platelet-derived growth factor (PDGF), fibroblast growth factors (FGFs), insulin-like growth factors (IGFs), nerve growth factor (NGF), vascular endothelial growth factor (VEGF), hepatocyte growth factor (HGF), transforming growth factors (TGFs), colony-stimulating factors (CSFs), erythropoietin (EPO), granulocyte colony-stimulating factor (G-CSF), granulocyte-macrophage colony-stimulating factor (GM-CSF), macrophage colony-stimulating factor (M-CSF), interleukins (some interleukins have growth factor activity), bone morphogenetic proteins (BMPs), growth differentiation factor (GDF), glial cell line-derived neurotrophic factor (GDNF), ciliary neurotrophic factor (CNTF), brain-derived neurotrophic factor (BDNF), neurotrophins (NTs), keratinocyte growth factor (KGF), and thrombopoietin (TPO)).Examples of the protein also include serum albumin, ovalbumin, casein, lysozyme, lipase, and the like. These proteins may be used alone or in a combination of two or more. Examples of the protein include protein complexes. Examples of the protein complexes include antibodies or antigen-binding fragments thereof.

Examples of nucleic acids include nucleic acids encoding any one or more of the proteins described above (DNA or mRNA), siRNA, miRNA, shRNA, and antisense oligonucleotides.

Examples of polysaccharides include LM pectin, HM pectin, hydroxypropylmethylcellulose phthalate, heparin, alginic acid, and carboxymethylcellulose. These polysaccharides may be used alone or in a combination of two or more.

As the lipid, an amphiphilic lipid (e.g., having a hydrophobic moiety and a hydrophilic moiety and exhibiting properties as a surfactant) can be preferably used. The lipid is preferably a dermatologically or pharmaceutically acceptable lipid. Such lipids are as described above. In certain embodiments, the lipid may be a nonionic lipid having a hydrophilic moiety. In certain embodiments, the lipid is an ionic lipid. In certain embodiments, the lipid is a mixture of a cationic lipid and an anionic lipid. In this embodiment, the mixture may preferably be a 1:1 mixture of a cationic lipid and an anionic lipid. In certain preferred embodiments, the mixture is a liquid. As cationic lipids, for example, the cationic lipids as described above can be used, and as anionic lipids, for example, the anionic lipids as described above can be used. In certain preferred embodiments, the cationic lipid comprises EDMPC+, and the anionic lipid may comprise one or more lipids selected from the group consisting of linoleic acid (Lin), oleic acid (Ole), and stearic acid (Ste). When the mixture is liquid, the production process is simplified. Specifically, when the mixture is liquid, lipid nanoparticles encapsulating the molecule of interest can be obtained simply by mixing a solution (particularly an aqueous solution) containing the molecule of interest and an ethanol solution containing the liquid lipid composition. In this case, the emulsification process and the subsequent solvent removal are unnecessary. From this manufacturing advantage, the lipid is preferably a liquid lipid composition. By reducing the mixing ratio of ethanol, a complex composition with a reduced ethanol content (e.g., an ethanol concentration of 5% or less, 4% or less, 3% or less, 2% or less, 1% or less, 0.5% or less, 0.4% or less, 0.3% or less, 0.2% or less, or 0.1% or less) can be obtained. If necessary, the ethanol concentration may be further reduced by solvent exchange using various techniques such as column chromatography or dialysis.

Examples of the molecule of interest include polypeptides. The polypeptide may be linear or cyclic. The polypeptide is not particularly limited, but may have a molecular weight of, for example, approximately 1 kDa to 500 kDa, 1 kDa to 400 kDa, 1 kDa to 300 kDa, 1 kDa to 200 kDa, for example, 5 kDa to 160 kDa, or 10 kDa to 100 kDa. The polypeptide may be, for example, a major histocompatibility complex (MHC) class I-restricted or class II-restricted polypeptide. The polypeptide may also be an enzyme (e.g., a metabolic enzyme). The polypeptide may also be a mitochondrial protein. The polypeptide may also be a cytoplasmic protein. The polypeptide may also be a nuclear protein. Cyclic polypeptides (e.g., 600 to 6,000 Da) and intracellular antibodies (e.g., scFv fragments of antibodies, approximately 30 kDa) can exert a desired physiological effect by binding to a target molecule within a cell. For example, cyclic polypeptides and intracellular antibodies are expected to have effects such as inhibiting protein-protein interactions (PPI) by a target molecule; by introducing them into a cell, they can inhibit intracellular PPI and provide pharmaceutical utility. Cyclic polypeptides and intracellular antibodies can also provide pharmaceutical utility by inducing the degradation of a target molecule through the attachment of a degradation-inducing tag (such as a degron). Therefore, the molecule of interest may be a polypeptide (preferably a cyclic polypeptide and an intracellular antibody) in the form of a fusion protein with a degradation-inducing tag.

Examples of the molecule of interest include antibodies. Antibodies include full-length antibodies. The antibody may be a polyclonal antibody or a monoclonal antibody. The antibody is preferably a human chimeric antibody, a humanized antibody, or a human antibody. The antibody is not particularly limited, and currently marketed antibodies can be used. Antibodies include monospecific antibodies and multispecific antibodies. The antibody is preferably a monoclonal antibody. Examples of antibodies include an antibody selected from the group consisting of muromonab-cd3, ibritumomab tiuxetan, iodine 131 tositumomab, catumaxomab, blinatumomab, moxetumomab pasudotox, abciximab, rituximab, basiliximab, infliximab, cetuximab, brentuximab vedotin, siltuximab, dinutuximab, obiltoxaximab, isatuximab, margetuximab, cetuximab sarotalocin sodium, daclizumab, palivizumab, trastuzumab, gemtuzumab ozogamicin, alemtuzumab, omalizumab, efalizumab, bevacizumab, natalizumab, tocilizumab, ranibizumab, eculizumab, certolizumab pegol, mogamulizumab, pertuzumab, trastuzumab emtansine, obinutuzumab, vedolizumab, pembrolizumab, idarucizumab, mepolizumab, elotuzumab, ixekizumab, reslizumab, atezolizumab, ocrelizumab, inotuzumab ozogamicin, emicizumab, benralizumab, galcanezumab, fremanezumab, tildrakizumab, caplacizumab, ibalizumab, ravulizumab, romosozumab, risankizumab, polatuzumab vedotin, romosozumab, brolucizumab, crizanlizumab, trastuzumab deruxtecan, eptinezumab, sacituzumab govitecan, inebilizumab, tafasitamab, belantamab mafodotin, satralizumab, naxitamab, loncastuximab tesirine, dostarlimab, bimekizumab, sutimlimab, adalimumab, panitumumab, golimumab, ustekinumab, canakinumab, ofatumumab, denosumab, ipilimumab, belimumab, raxibacumab, ramucirumab, nivolumab, secukinumab, evolocumab, alirocumab, necitumumab, daratumumab, brodalumab, olaratumab, bezlotoxumab, avelumab, durvalumab, dupilumab, bezlotoxumab, guselkumab, sarilumab, burosumab, erenumab, lanadelumab, emapalumab, enfortumab vedotin, teprotumumab, atoltivimab, maftivimab, odesivimab, ansuvimab, evinacumab, amivantamab, aducanumab, tralokinumab, anifrolumab, tisotumab vedotin, tezepelumab, casirivimab + imdevimab, regdanvimab, sotrovimab, cemiplimab, faricimab, and tebentafusp, or an antibody having the three complementarity-determining regions (CDRs) of the heavy chain variable region and the three complementarity-determining regions (CDRs) of the light chain variable region thereof, or an antigen-binding fragment thereof. Examples of antibodies also include antibodies that bind to intracellular factors (e.g., intracellular proteins, cytoplasmic proteins). Antibody fragments include, for example, Fab, Fab', F(ab')₂, and scFv. The antibody can bind to an intracellular factor and directly or indirectly inhibit or activate the activity of the intracellular factor, or neutralize the interaction between the intracellular factor and another factor to inhibit or activate the function of the intracellular factor. Therefore, the antibody or antigen-binding fragment thereof can preferably directly or indirectly inhibit or activate the activity of an intracellular factor. Further, the antibody or antigen-binding fragment thereof can preferably neutralize the interaction between the intracellular factor and another factor.

Examples of the molecule of interest include nucleic acids. Examples of the nucleic acids include DNA, RNA, and modified products thereof (i.e., modified nucleic acids). Examples of the DNA include linear or cyclic single-stranded DNA, and linear or cyclic double-stranded DNA. Examples of the RNA include, for example, linear single-stranded RNA and linear double-stranded RNA. For example, examples of the RNA include mRNA, microRNA (miRNA), siRNA, shRNA, tRNA, aptamers, and rRNA. Examples of the nucleic acids also include antisense oligos. Antisense oligos generally contain DNA and/or RNA and have a sequence complementary to a target nucleic acid. siRNA and shRNA have an appropriate length (e.g., 20 to 25-mer) and an appropriate sequence (complementarity with a target sequence) for knocking down a target nucleic acid. The nucleic acids may contain modified nucleic acids. The length of the siRNA may be, for example, about 20 to about 30 bases, about 20 to about 24 bases, or about 21 to about 23 bases. Examples of the nucleic acids also include gapmers and mixmers. A gapmer is a single-stranded nucleic acid having a DNA portion and an RNA or modified nucleic acid portion, wherein the RNA or modified nucleic acid portion is located at both ends of the DNA portion. The gapmer may be, for example, a double-stranded nucleic acid. A mixmer is a nucleic acid containing an antisense that includes different types of nucleic acids. The nucleic acid may also be a vector (e.g., an expression vector) encoding any of the above. MicroRNAs are RNA molecules about 21 to about 25 nucleotides in length. Typically, they suppress translation by binding to mRNA or regulate the expression level by degrading mRNA. The expression vector has a gene expression cassette containing the above nucleic acid operably linked to a regulatory sequence. The expression vector may be a cyclic vector (e.g., a plasmid vector). In a preferred embodiment where the molecule of interest is a nucleic acid, the lipid may be a non-ionic lipid, or more preferably, a cationic lipid or a mixture of a cationic lipid and an anionic lipid (e.g., a liquid lipid composition that is a 1:1 mixture). In a preferred embodiment where the molecule of interest is a nucleic acid, the cationic lipid may also be an ionizable lipid. In a preferred embodiment where the molecule of interest is a nucleic acid, the first complex may be a liposome encapsulating the nucleic acid, rather than a mere complex of the nucleic acid and the cationic lipid.

Examples of the liposome include unilamellar liposomes, multilamellar liposomes (particularly those having one or more lipid bilayers), stealth liposomes (e.g., those whose surfaces are coated with uncharged hydrophilic molecules or polymers such as PEG to evade the immune system or improve blood circulation retention compared to those without such coating), immunoliposomes (those having cancer antigen-targeting molecules such as antibodies or peptides linked to the surface), and thermo-sensitive liposomes (those that release encapsulated components in a heat-responsive manner), any of which can be advantageously used in the present invention. Coating the surface with an anti-CD71 antibody may also promote permeability across the blood-brain barrier.

Lipids for liposome formation are not particularly limited, but examples thereof include phosphatidylcholine, phosphatidylethanolamine, phosphatidylserine, phosphatidylglycerol, PEG-modified lipids (e.g., PEG-DSPE (1,2-distearoyl-sn-glycero-3-phosphoethanolamine-polyethylene glycol)), cationic lipids (e.g., DOTAP (1,2-dioleoyl-3-trimethylammonium-propane)), and sphingomyelin. In addition, other pharmaceutically acceptable lipids may be used.

Proteins encoded by the mRNA include components specific to heterologous organisms, such as pathogens, or portions thereof, and components specific to cancer cells or portions thereof. Pathogens include pathogenic viruses, pathogenic microorganisms, and pathogenic parasites. Pathogenic viruses include viruses selected from the group consisting of human immunodeficiency virus (HIV), hepatitis A, hepatitis B, hepatitis C, herpesvirus, adenovirus, influenza virus, flavivirus, echovirus, rhinovirus, coxsackievirus, coronavirus (e.g., severe acute respiratory syndrome-related coronavirus (SARS-CoV), Middle East respiratory syndrome-related coronavirus (MERS), SARS-CoV-2), respiratory syncytial virus, mumps virus, rotavirus, measles virus, rubella virus, parvovirus, vaccinia virus, human T-cell leukemia (HTL) virus, dengue virus, papillomavirus, molluscum contagiosum virus, poliovirus, rabies virus, John Cunningham (JC) virus, and arboviral encephalitis virus. Pathogenic microorganisms include pathogenic bacteria, and examples of the pathogenic bacteria include bacteria selected from the group consisting of *Chlamydia, Rickettsiales,* Mycobacteria, Staphylococci, Streptococci, Pneumococci, Meningococci and Gonococci, Klebsiella, Proteus, Serratia, Pseudomonas, Legionella, Diphtheria, Salmonella, Bacilli, Cholera, Tetanus, Botulism, Anthrax, Plague, Leptospirosis, and Lyme disease bacteria. Pathogenic microorganisms include pathogenic fungi, and examples of the pathogenic fungi include pathogenic fungi selected from the group consisting of Candida (e.g., albicans, krusei, glabrata, tropicalis), Cryptococcus neoformans, Aspergillus (e.g., fumigatus, niger), Mucorales (e.g., Mucor, Absidia, Rhizopus), Sporothrix schenckii, Blastomyces dermatitidis, Paracoccidioides brasiliensis, Coccidioides immitis, and Histoplasma capsulatum. Pathogenic parasites include pathogenic parasites selected from the group consisting of Entamoeba histolytica, Balantidium coli, Naegleria fowleri, Acanthamoeba, Giardia lamblia, Cryptosporidium, Pneumocystis carinii, Plasmodium vivax, Babesia microti, Trypanosoma brucei, Trypanosoma cruzi, Leishmania donovani, Toxoplasma gondii, and Ancylostoma braziliense. Accordingly, in an embodiment of the present invention, the mRNA can encode a constituent component (preferably a surface antigen) of these pathogens or a portion thereof. In particular, among surface antigens of pathogens, an antigen utilized by a pathogen for infecting cells, such as the S protein of betacoronavirus, can be preferably encoded by the mRNA of the present invention. Cancers include cancers selected from the group consisting of bladder cancer, breast cancer, uterine cancer, endometrial cancer, ovarian cancer, colorectal cancer, colon cancer, head and neck cancer, lung cancer, stomach cancer, germ cell cancer, bone cancer, squamous cell carcinoma, skin cancer, central nervous system neoplasms, lymphoma, leukemia, sarcoma, virus-related cancer, small cell lung cancer, non-small cell lung cancer, gastrointestinal cancer, Hodgkin's or non-Hodgkin's lymphoma, pancreatic cancer, glioblastoma, glioma, cervical cancer, ovarian cancer, liver cancer, myeloma, salivary gland cancer, kidney cancer, basal cell carcinoma, melanoma, prostate cancer, vulvar cancer, thyroid cancer, testicular cancer, esophageal cancer, and head and neck cancer. In an embodiment of the present invention, the mRNA may be an antigen specific to these cancers (cancer-specific antigen, preferably a cancer-specific surface antigen) or a portion thereof. The cancer-specific antigen may be, for example, a neoantigen or a portion thereof characteristic of a neoantigen. Cancer-specific antigens also include, for example, cancer-testis antigens (e.g., MAGE family proteins, NY-ESO-1), differentiation antigens (e.g., melanoma tyrosine kinase, Melan-A/MART-1, prostate cancer PSA), overexpressed cancer antigens (e.g., Her-2/Neu, survivin, telomerase, and WT-1), oncogenic variants of β-catenin, oncogenic variants of CDK4, and MUC-1, particularly MUC-1 having an altered glycosylation pattern, and human papillomavirus E6 or E7. Regarding these, a person skilled in the art can appropriately select an antigen and prepare mRNA suitable for the present invention according to the infectious disease or the type of cancer from which a subject suffers and the type of antigen expressed in the cancer. mRNA for naturally occurring mutant proteins can also be appropriately designed.

In an embodiment, the molecule of interest is mRNA, and the mRNA is a cancer antigen. In this embodiment, a complex of the molecule of interest, a lipid, and an extracellular vesicle can be administered to a subject having cancer. The subject who has received the administration may be administered an immune checkpoint inhibitor. As the immune checkpoint inhibitor, for example, a checkpoint inhibitor that blocks signals mediated by PD-1 or a checkpoint inhibitor that blocks signals mediated by CTLA-4 can be used. Examples of the immune checkpoint inhibitor include antibodies capable of neutralizing the binding between PD-1 and PD-L1 or PD-L2, and antibodies capable of neutralizing the binding between CTLA-4 and CD80 or CD86. Examples of the antibodies capable of neutralizing the binding between PD-1 and PD-L1 include anti-PD-1 antibodies and anti-PD-L1 antibodies capable of neutralizing the binding between PD-1 and PD-L1. Examples of the antibodies capable of neutralizing the binding between PD-1 and PD-L2 include anti-PD-1 antibodies and anti-PD-L2 antibodies capable of neutralizing the binding between PD-1 and PD-L2. Examples of the antibodies capable of neutralizing the binding between CTLA-4 and CD80 or CD86 include anti-CTLA-4 antibodies capable of neutralizing the binding between CTLA-4 and CD80 or CD86. The immune checkpoint inhibitor is not particularly limited but includes, for example, anti-PD-1 antibodies such as nivolumab, pembrolizumab, and pidilizumab; anti-PD-L1 antibodies such as atezolizumab, durvalumab, and avelumab; anti-CTLA-4 antibodies such as ipilimumab; anti-TIM-3 antibodies such as TSR-022 (WO 2016/161270) and MBG453 (WO 2015/117002); anti-LAG-3 antibodies such as LAG525 (US 2015/0259420); anti-TIGIT antibodies such as MAB10 (WO 2017/059095); anti-BTLA antibodies such as BTLA-8.2 (The Journal of Clinical Investigation, 2010, Vol. 120, No. 1, pp. 157-167); and anti-VISTA antibodies such as JNJ-61610588 (WO 2016/207717). In a preferred embodiment, the immune checkpoint inhibitor is an anti-PD-1 antibody or an anti-PD-L1 antibody, more preferably an anti-PD-1 antibody or an anti-PD-L1 antibody capable of neutralizing the binding between PD-1 and PD-L1, and even more preferably an anti-PD-1 antibody selected from the group consisting of nivolumab, pembrolizumab, and pidilizumab, or one or more anti-PD-L1 antibodies selected from the group consisting of atezolizumab, durvalumab, and avelumab.

In an embodiment, at least one or more uridines in the mRNA may be replaced with pseudouridines. The pseudouridine may be 1-methylpseudouridine. The mRNA may be one transcribed from cDNA; that is, it may not have an intron. Further, the mRNA may have a Cap structure at the 5' end (Furuichi Y & Miura K. Nature. 1975; 253(5490): 374-5). Regarding the Cap structure, a CapO structure can be added to mRNA by the Anti-Reverse Cap Analogue (ARCA) method using a Cap analog (Stepinski J et al. RNA. 2001 Oct; 7(10): 1486-95). The CapO structure of the mRNA can be converted to a Cap1 structure by further performing 2'-O-methyltransferase treatment. These can be performed by conventional methods, for example, using commercially available kits such as ScriptCap m7G Capping System and ScriptCap 2'-O-Methyltransferase Kit, or T7 mScript Standard mRNA Production System (AR Brown CO., LTD). The mRNA can have a poly(A) tail. The addition of the poly(A) tail can be performed by a conventional method, for example, using A-Plus Poly(A) Polymerase Tailing Kit (AR Brown CO., LTD). Therefore, in an embodiment, the mRNA may be an mRNA having a Cap structure at the 5' end and a poly(A) tail at the 3' end, and preferably having at least a portion of uridines replaced with pseudouridines (preferably 1-methylpseudouridines). The mRNA may be an isolated mRNA or a synthesized mRNA.

In an embodiment, the molecule of interest is an RNP (ribonucleoprotein). The RNP is not particularly limited but may be, for example, a complex of a Cas endonuclease of the CRISPR/Cas system and a guide RNA (gRNA). The Cas endonuclease has a size of about 130 kDa, and the RNP complex has a size of about 180 kDa, which is a size that can be sufficiently encapsulated within an exosome. The gRNA can include a crRNA and a tracrRNA, and the crRNA and the tracrRNA may be linked by a hairpin to form a single-guide RNA (sgRNA).

Cas endonucleases are broadly classified into Class 1, Class 2, and others. Examples of Class 1 include E. coli-derived Cas nucleases such as Cascade (CRISPR-associated complex for antiviral defense), Cas3 nuclease, and Csm/Cmr nuclease. Examples of Class 2 include Cas9 endonuclease derived from Staphylococcus aureus, Streptococcus pyogenes, or Neisseria meningitidis, Cas12a, Cas13, Cas14, and Casφ. Some nucleases include an RNA-protein complex. The Cas endonuclease may be a nickase. A nickase is, for example, one in which either the RuvC domain or the HNH domain of Cas9 is inactivated, and can introduce a single-strand break into a target sequence.

The gRNA is an RNA capable of forming a complex with a Cas protein and guiding the Cas protein to a target region. The crRNA includes a spacer region having a sequence complementary to the nucleotide sequence of the target region and a repeat region having a sequence complementary to a tracrRNA. The tracrRNA has an anti-repeat region having a sequence complementary to the repeat region and a 3' tail region that forms a complex with a Cas nuclease. Typically, a protospacer adjacent motif (PAM) is required in a region adjacent to the 3' side of the cleavage region. Therefore, the spacer region of the gRNA can be designed toward a sequence adjacent to the 5' side of the PAM. A person skilled in the art can design the gRNA using well-known and conventional techniques.

When a Cas-gRNA complex is encapsulated in an exosome, the exosome can be contacted with a target cell in vivo or in vitro to target a target region of the genome within the target cell, or thereby cleave the target region, or thereby modify the target region. Examples of the modification include introduction of indels (insertions or deletions), insertion of a target nucleotide sequence, and nucleotide substitutions. These can be realized, for example, by causing the Cas-gRNA complex to target a target region of the genome in the presence of a template DNA. The template DNA comprises an upstream homology arm and a downstream homology arm, and a sequence may or may not exist between the upstream homology arm and the downstream homology arm. The upstream homology arm and the downstream homology arm have sequences (particularly complementary sequences) capable of homologous recombination with the upstream and downstream of the target region. When the Cas-gRNA complex is caused to target the target region of the genome in the presence of the template DNA, the genomic sequence between the upstream and downstream of the target region is replaced with the sequence between the upstream homology arm and the downstream homology arm of the template DNA. It is practical to also encapsulate the template DNA in the exosome, and due to high loading efficiency, many exosomes can encapsulate the template DNA together with the RNP.

As used herein, a "control sequence" is a sequence having an activity to drive a gene operably linked thereto and transcribe RNA from the gene. The control sequence is, for example, a promoter. Examples of the promoter include Class I promoters (which can be used for transcription of rRNA precursors), Class II promoters (which include a core promoter and upstream promoter elements and can be used for transcription of mRNA), and Class III promoters (further classified into types I, II, and III). The control sequence is also called a regulatory sequence and may be any promoter capable of transcribing mRNA in cells such as animal cells or plant cells. For example, various pol II promoters can be used as the first control sequence. Examples of pol II promoters include, but are not limited to, CMV promoter, EF1 promoter (EF1α promoter), SV40 promoter, MSCV promoter, hTERT promoter, β-actin promoter, CAG promoter, and CBh promoter. Further, the promoter can also include promoters that drive bacteriophage-derived RNA polymerases such as T7 promoter, T3 promoter, and SP6 promoter, and pol III promoters such as U6 promoter. The T7 promoter is preferably used for transcription from cyclic DNA, and the SP6 promoter is preferably used for transcription from linear DNA.

Examples of the molecule of interest include low-molecular-weight compounds (small molecule compounds). Examples of the low-molecular-weight compounds include antioxidants (e.g., ascorbic acid, isoascorbic acid, vitamin E, catechin, dibutylhydroxytoluene, tocopherol, butylhydroxyanisole, polyphenols, resveratrol (resveratrol 3,5,4'-trihydroxy-trans-stilbene), coenzyme Q10, N-acetylcysteine, 2,2,6,6-tetramethylpiperidine 1-oxyl (TEMPO), superoxide dismutase (SOD), nicotinamide mononucleotide (NMN), astaxanthin, β-carotene, and glutathione). Examples of the low-molecular-weight compounds include anti-tumor agents. Examples of the anti-tumor agents include paclitaxel, actinomycin, camptothecin, irinotecan, epirubicin, etoposide, oxaliplatin, L-asparaginase, alectinib, pemetrexed, ifosfamide, gefitinib, cyclophosphamide, carboplatin, cabazitaxel, imatinib, capecitabine, amrubicin, gemcitabine, crizotinib, cisplatin, afatinib, regorafenib, cytarabine, sunitinib, lapatinib, erlotinib, docetaxel, tegafur-uracil, temozolomide, liposomal doxorubicin, dacarbazine, dasatinib, vinorelbine, nimustine, nedaplatin, sorafenib, nogitecan, eribulin, vincristine, bleomycin, vinblastine, fluorouracil, methotrexate, and mitomycin. Examples of the low-molecular-weight compounds include those having bioactivity. Examples of the low-molecular-weight compounds include water-soluble compounds or water-insoluble compounds.

A complex of a molecule of interest and a lipid can be obtained by mixing the molecule of interest and the lipid. Although the mixing is performed under conditions suitable for complex formation, the complex formation is usually promoted simply by mixing. The complex usually includes micelles formed by the lipid. Preferably, the molecule of interest is encapsulated in the micelles; for example, the molecule of interest interacts with a hydrophilic portion and/or a hydrophobic portion of the lipid constituting the micelles. In a preferred embodiment, the lipid is an ionic lipid or a mixture thereof, and may be a non-ionic lipid or may contain a non-ionic lipid, more preferably is a cationic lipid or contains a cationic lipid, even more preferably is a mixture of a cationic lipid and an anionic lipid, and still more preferably is a 1:1 mixture of a cationic lipid and an anionic lipid. Mixing is performed under suitable conditions.

The complex of the molecule of interest and the lipid may be a lipid nanoparticle (LNP). As used herein, "lipid nanoparticle" (LNP) means a nanovesicle composed of lipid molecules. Examples of the lipid nanoparticle include the nucleic acid-lipid particles described in US 8,058,069 B, which is incorporated herein by reference in its entirety. Amphiphilic lipids can form lipidic nanoparticles in an aqueous solution. A person skilled in the art can appropriately select a lipid and cause the lipid to form lipidic nanoparticles. Lipid nanoparticles are used for delivering nucleic acids such as single-stranded nucleic acids and double-stranded nucleic acids into cells, and can also be preferably used for delivering nucleic acids to extracellular vesicles.

The lipid nanoparticle may contain one or more, or preferably all, selected from the group consisting of cationic lipids, non-cationic lipids (or neutral lipids), sterols, and PEG lipids. In an embodiment, the cationic lipid is an ionizable cationic lipid. In an embodiment, the non-cationic lipid is a neutral lipid. In an embodiment, the sterols include cholesterol.

In an embodiment, examples of the cationic lipid include 2,2-dilinoleyl-4-dimethylaminoethyl-[1,3]-dioxolane (DLin-KC2-DMA), dilinoleyl-methyl-4-dimethylaminobutyrate (DLin-MC3), [(4-hydroxybutyl)azanediyl]di(hexane-6,1-diyl) bis(2-hexyldecanoate) (ALC-0315), 9-heptadecanyl 8-{(2-hydroxyethyl)[6-oxo-6-(undecyloxy)hexyl]amino}octanoate (SM-102), di((Z)-non-2-en-1-yl) 9-((4-(dimethylamino)butanoyl)oxy)heptadecanedioate (L319), (12Z,15Z)-N,N-dimethyl-2-nonylhenicosa-12,15-dien-1-amine (L608), and N,N-dimethyl-1-[(1S,2R)-2-octylcyclopropyl]heptadecan-8-amine (L530). In an embodiment, the cationic lipid may contain L608, or may be L608. In an embodiment, the cationic lipid may contain L530, or may be L530.

In certain embodiments, the cationic lipid may be 2-amino-3-[(9Z,12Z)-octadeca-9,12-dien-1-yloxy]-2-{[(9Z,2Z)-octadeca-9,12-dien-1-yloxy]methyl}propan-1-ol (Compound 1 of US20130150625); 2-amino-3-[(9Z)-octadec-9-en-1-yloxy]-2-{[(9Z)-octadec-9-en-1-yloxy]methyl}propan-1-ol (Compound 2 of US20130150625); 2-amino-3-[(9Z,12Z)-octadeca-9,12-dien-1-yloxy]-2-[(octyloxy)methyl]propan-1-ol (Compound 3 of US20130150625); and 2-(dimethylamino)-3-[(9Z,12Z)-octadeca-9,12-dien-1-yloxy]-2-{[(9Z,12Z)-octadeca-9,12-dien-1-yloxy]methyl}propan-1-ol (Compound 4 of US20130150625); or any pharmaceutically acceptable salt or stereoisomer thereof.

In certain embodiments, ionizable cationic lipids (also referred to as ionizable lipids) may be 2,2-dilinoleyl-4-dimethylaminoethyl-[1,3]-dioxolane (DLin-KC2-DMA), dilinoleyl-methyl-4-dimethylaminobutyrate (DLin-MC3-DMA), or di((Z)-non-2-en-1-yl) 9-((4-(dimethylamino)butanoyl)oxy)heptadecanedioate (L319).

In certain embodiments, neutral lipids include 1,2-distearoyl-sn-glycero-3-phosphocholine (DSPC), dipalmitoylphosphatidylcholine (DPPC), PO phosphatidylcholine (POPC), 1,2-dioleoyl-sn-glycero-3-phosphoethanolamine (DOPE), and sphingomyelin (SM). The neutral lipid may be neutral as a whole molecule under neutral pH conditions.

Examples of the sterol include animal-derived sterols such as cholesterol, cholesterol succinate, lanosterol, dihydrolanosterol, desmosterol, and dihydrocholesterol; plant-derived sterols (phytosterols) such as stigmasterol, sitosterol, campesterol, and brassicasterol; and microorganism-derived sterols such as zymosterol and ergosterol. The lipid nanoparticle preferably contains a sterol, and more preferably contains cholesterol.

The lipid nanoparticle may comprise a lipid having a polyalkylene glycol modification. Examples of polyalkylene glycols include polyethylene glycol, polypropylene glycol, polytetramethylene glycol, and polyhexamethylene glycol. The weight-average molecular weight of the polyalkylene glycol is, for example, about 300 to 10,000, preferably about 500 to 10,000, and more preferably about 1,000 to 5,000.

As the modification of lipids with polyethylene glycol, stearylated polyethylene glycol (e.g., stearic acid PEG45 (STR-PEG45), etc.) can be used. In addition, as PEG lipids, polyethylene glycol derivatives such as N-[carbonyl-methoxypolyethylene glycol-2000]-1,2-dipalmitoyl-sn-glycero-3-phosphoethanolamine, n-[carbonyl-methoxypolyethylene glycol-5000]-1,2-dipalmitoyl-sn-glycero-3-phosphoethanolamine, N-[carbonyl-methoxypolyethylene glycol-750]-1,2-distearoyl-sn-glycero-3-phosphoethanolamine, N-[carbonyl-methoxypolyethylene glycol-2000]-1,2-distearoyl-sn-glycero-3-phosphoethanolamine, N-[carbonyl-methoxypolyethylene glycol-5000]-1,2-distearoyl-sn-glycero-3-phosphoethanolamine, and 1,2-dimyristoyl-rac-glycero-3-methoxypolyethylene glycol-2000 (PEG-DMG) can also be used.

The lipid nanoparticle preferably has an average particle size of 400 nm or less, and more preferably has an average particle size of 300 nm or less. The average particle size of the lipid nanoparticle means a number-average particle size measured by a dynamic light scattering (DLS) method. The measurement by the dynamic light scattering method can be performed by a conventional method using a commercially available DLS apparatus or the like. The polydispersity index (PDI) of the lipid nanoparticle may be about 0.05 to 0.5, about 0.05 to 0.4, about 0.05 to 0.3, preferably about 0.05 to 0.2, more preferably about 0.05 to 0.15, or about 0.1. The lipid nanoparticle can be prepared using a microfluidic device according to a conventional method. Further, it is easy to control the PDI of the LNP to about 0.1 using a conventional method.

In certain embodiments, the lipid nanoparticle may comprise about 20 mol% to about 60 mol% cationic lipid, about 0.5 mol% to about 15 mol% PEG-modified lipid, about 25 mol% to about 55 mol% sterol, and about 20 mol% to about 60 mol% non-cationic lipid. In certain preferred embodiments, the lipid nanoparticle comprises about 45 mol% to about 50 mol% cationic lipid, about 9 to 10 mol% non-cationic lipid (preferably neutral lipid), about 35 mol% to about 45 mol% sterol (preferably cholesterol), and about 1.5 mol% to about 2 mol% PEG lipid.

The lipid nanoparticle may have a positive charge in an aqueous solution. The lipid nanoparticle may also have a negative charge in an aqueous solution. The lipid nanoparticle may also be uncharged in an aqueous solution. In certain preferred embodiments, the lipid nanoparticle has a positive charge in an aqueous solution.

The oily base is not particularly limited, but may be, for example, sterol derivatives such as cholesteryl oleate, cholesteryl linoleate, cholesteryl myristate, cholesteryl palmidate, and cholesteryl arachidate, or long-chain fatty acid esters such as isopropyl myristate (IPM), octyldodecyl myristate, cetyl myristate, ethyl oleate, ethyl linoleate, isopropyl linoleate, isopropyl palmitate, and butyl stearate. The oily phase may also be carboxylic acid esters such as ethyl lactate, cetyl lactate, triethyl citrate, diisopropyl adipate, diethyl sebacate, diisopropyl sebacate, and cetyl 2-ethylhexanoate, or hydrocarbons such as petrolatum, paraffin squalane, and vegetable squalane, or silicones. The oily base may be used alone or in a mixture of two or more.

The aqueous base is not particularly limited, but examples thereof include water, distilled water, Milli-Q water, and physiological saline. The aqueous base may contain pharmaceutically acceptable additives. Examples of pharmaceutically acceptable additives include salts, pH adjusters, thickeners, colorants, surfactants, chelating agents, solubilizers, preservatives, isotonizing agents, and stabilizers.

The complex of the molecule of interest and the lipid can be mixed with extracellular vesicles. Thereby, a complex (second complex) of the molecule of interest, the lipid, and the extracellular vesicles, and a composition comprising the same are obtained. Mixing is performed under conditions suitable for complex formation, but usually, mere admixture promotes the complex formation. The mixing ratio of the complex to the extracellular vesicles can be appropriately determined. The mixing ratio can be, for example, 1:1000 to 1000:1, 1:100 to 100:1, 1:50 to 50:1, 1:30 to 30:1, 1:20 to 20:1, 1:10 to 10:1, 1:5 to 5:1, or 1:2 to 2:1 (about 1:1). In a preferred embodiment, the lipid is an ionic lipid or a mixture thereof, more preferably a mixture of a cationic lipid and an anionic lipid, and even more preferably a 1:1 mixture of a cationic lipid and an anionic lipid. Usually, in this complex, the extracellular vesicles and the complex of the molecule of interest and the lipid are fused to form a vesicle, and the molecule of interest is encapsulated in the vesicle; for example, the molecule of interest interacts with the hydrophilic moiety and/or the hydrophobic moiety of the lipid constituting the vesicle. By doing so, it becomes possible to introduce the molecule of interest into extracellular vesicles. Introduction of the molecule of interest into extracellular vesicles can contribute to making the extracellular vesicles highly functional. Mixing is performed under suitable conditions.

The complex of a molecule of interest, a lipid, and an extracellular vesicle can introduce the molecule of interest into a cell by being contacted with the cell. Therefore, it is desirable that the molecule of interest has an activity to regulate the physiological activity of the cell. The contact is performed under suitable conditions.

The first complex effectively delivers the molecule of interest to the brain, lung, kidney, muscle (e.g., myocardium), and spleen. Therefore, the first complex can be used for delivering the molecule of interest to any of these tissues.
The second complex delivers the molecule of interest to the brain, kidney, muscle (e.g., myocardium), ovary, pancreas, and spleen. Therefore, the second complex can be used for delivering the molecule of interest to any of these tissues.

Therefore, the first complex can be used to treat diseases by delivering the molecule of interest that provides a therapeutic benefit to the respective organ or tissue. The second complex can also be used to treat the above diseases by delivering the molecule of interest that provides a therapeutic benefit to the respective organ or tissue. The second complex is particularly easily introduced into cells and may be more beneficial for treatment of the above diseases. Therefore, the molecule of interest may be a molecule that functions intracellularly.

**In** neurodegenerative diseases, the accumulation of abnormal proteins within brain tissue or neurons is considered the pathogenesis. For example, accumulation of amyloid β or tau is observed in Alzheimer's disease; accumulation of α-synuclein is observed in Parkinson's disease and Dementia with Lewy Bodies; accumulation of polyglutamine is observed in polyglutamine diseases (e.g., Huntington's disease and spinocerebellar ataxia); accumulation of TDP-43 or SOD1 is observed in amyotrophic lateral sclerosis; and accumulation of tau or TDP-43 is observed in frontotemporal dementia. An antibody that binds to and removes any appropriate one of these can produce a therapeutic benefit for the above diseases. As the molecule of interest, an antibody or an antigen-binding fragment thereof that binds to the above proteins or abnormal proteins, particularly an intracellular antibody (e.g., scFv), can be used. Degradation of a target protein can be induced by attaching a protein degradation-promoting signal, such as a chaperone-mediated autophagy (CMA) signal, to the intracellular antibody.

According to the present disclosure, provided is a method for treating a disease in a subject having the disease that would benefit from administration, the method comprising administering an effective amount of the complex or composition of the present disclosure to the subject. According to the present disclosure, the complex or composition of the present disclosure is provided for use in the subject in the above method. According to the present disclosure, provided is the use of the complex or composition of the present disclosure in the manufacture of a medicament for use in the subject in the above method. As used herein, an "effective amount" means an amount sufficient to exert physiological relevance. The administration route is not particularly limited and includes, for example, transdermal administration, intradermal administration, subcutaneous administration, intravenous administration, intraperitoneal administration, eye drops, intravitreal administration, intramuscular administration, intramedullary administration, intracerebrospinal fluid administration, intrathecal administration, and intracardiac administration.

According to the present disclosure, the complex is obtained by a method comprising:
obtaining a complex of a molecule of interest and a lipid;
contacting the obtained complex with an extracellular vesicle to obtain a composite particle of the complex and the extracellular vesicle,
wherein the molecule of interest is encapsulated in the extracellular vesicle.

According to the present disclosure, there is also provided a method for introducing a molecule of interest into an extracellular vesicle, comprising:
obtaining a complex of the molecule of interest and a lipid mixture; and
contacting the obtained complex with an extracellular vesicle to obtain a composite particle of the complex and the extracellular vesicle,
whereby the molecule of interest is introduced into the composite particle.

According to the present disclosure, there is also provided a method for introducing a molecule of interest into an extracellular vesicle, comprising:
obtaining a complex of the molecule of interest and a nonionic lipid; and
contacting the obtained complex with an extracellular vesicle to obtain a composite particle of the complex and the extracellular vesicle,
whereby the molecule of interest is introduced into the composite particle.

According to the present disclosure, there is also provided a method for introducing a molecule of interest into an extracellular vesicle, comprising:
obtaining a complex of the molecule of interest and a cationic lipid; and
contacting the obtained complex with an extracellular vesicle to obtain a composite particle of the complex and the extracellular vesicle,
whereby the molecule of interest is introduced into the composite particle.

According to the present disclosure, there is also provided a method for introducing a molecule of interest into an extracellular vesicle, comprising:
obtaining a complex of the molecule of interest and a cationic lipid or an anionic lipid; and
contacting the obtained complex with an extracellular vesicle to obtain a composite particle of the complex and the extracellular vesicle,
whereby the molecule of interest is introduced into the composite particle.

According to the present disclosure, there is also provided a method for introducing a molecule of interest into an extracellular vesicle, comprising:
obtaining a complex of the molecule of interest, a cationic lipid, and an anionic lipid; and
contacting the obtained complex with an extracellular vesicle to obtain a composite particle of the complex and the extracellular vesicle,
whereby the molecule of interest is introduced into the composite particle.

According to the present disclosure, there is provided a method for introducing a molecule of interest into a cell, comprising:
obtaining a complex of the molecule of interest and a lipid mixture;
contacting the obtained complex with an extracellular vesicle to obtain a composite particle of the complex and the extracellular vesicle, whereby the molecule of interest is introduced into the composite particle; and
contacting the obtained composite particle with a cell to introduce the molecule of interest into the cell.

According to the present disclosure, there is provided a method for introducing a molecule of interest into a cell, comprising:
obtaining a complex of the molecule of interest and a nonionic lipid;
contacting the obtained complex with an extracellular vesicle to obtain a composite particle of the complex and the extracellular vesicle, whereby the molecule of interest is introduced into the composite particle; and
contacting the obtained composite particle with a cell to introduce the molecule of interest into the cell.

According to the present disclosure, there is provided a method for introducing a molecule of interest into a cell, comprising:
obtaining a complex of the molecule of interest and a cationic lipid;
contacting the obtained complex with an extracellular vesicle to obtain a composite particle of the complex and the extracellular vesicle, whereby the molecule of interest is introduced into the composite particle; and
contacting the obtained composite particle with a cell to introduce the molecule of interest into the cell.

According to the present disclosure, there is provided a method for introducing a molecule of interest into a cell, comprising:
obtaining a complex of the molecule of interest and a cationic lipid or an anionic lipid;
contacting the obtained complex with an extracellular vesicle to obtain a composite particle of the complex and the extracellular vesicle, whereby the molecule of interest is introduced into the composite particle; and
contacting the obtained composite particle with a cell to introduce the molecule of interest into the cell.

According to the present disclosure, there is provided a method for introducing a molecule of interest into a cell, comprising:
obtaining a complex of the molecule of interest, a cationic lipid, and an anionic lipid;
contacting the obtained complex with an extracellular vesicle to obtain a composite particle of the complex and the extracellular vesicle, whereby the molecule of interest is introduced into the composite particle; and
contacting the obtained composite particle with a cell to introduce the molecule of interest into the cell.

Regarding the above method, in an embodiment, the molecule of interest is a nucleic acid, and (i) the complex is a complex of the molecule of interest and a non-ionic lipid, and the complex particles are complex particles of the molecule of interest, the non-ionic lipid, and an extracellular vesicle; preferably, (ii) the complex is a complex of the molecule of interest and a cationic lipid, and the complex particles are complex particles of the molecule of interest and the cationic lipid; more preferably, (iii) the complex is a complex of the molecule of interest, a cationic lipid, and a non-ionic lipid, and the complex particles are complex particles of the molecule of interest, the cationic lipid, the non-ionic lipid, and an extracellular vesicle; and even more preferably, (iv) the complex is a complex of the molecule of interest, a cationic lipid, and an anionic lipid, and the complex particles are complex particles of the molecule of interest, the cationic lipid, the anionic lipid, and an extracellular vesicle. When the isoelectric point of the molecule of interest is high (for example, when the pI exceeds 7 or is 8 or more), an anionic lipid can be further included in the complexes and complex particles of the above (ii), (iii), or (iv). Since nucleic acids are polyanions, it is preferable to cause them to form a complex with an ionic lipid (particularly a cationic lipid, and a mixture of a cationic lipid and an anionic lipid).

With respect to the above methods, in certain embodiments, the molecule of interest is a protein (e.g., an antibody), and (i) the complex is a complex of the molecule of interest and a nonionic lipid, and the composite particle is a composite particle of the molecule of interest, a nonionic lipid, and an extracellular vesicle; preferably, (ii) the complex is a complex of the molecule of interest and a cationic lipid, and the composite particle is a composite particle of the molecule of interest and a cationic lipid; more preferably, (iii) the complex is a complex of the molecule of interest, a cationic lipid, and a nonionic lipid, and the composite particle is a composite particle of the molecule of interest, a cationic lipid, a nonionic lipid, and an extracellular vesicle; and still more preferably, (iv) the complex is a complex of the molecule of interest, a cationic lipid, and an anionic lipid, and the composite particle is a composite particle of the molecule of interest, a cationic lipid, an anionic lipid, and an extracellular vesicle. When the isoelectric point of the molecule of interest is high (e.g., when the pI exceeds 7, or is 8 or more), an anionic lipid can be further included in the complexes and composite particles of (ii), (iii), or (iv) above.

In an embodiment, the method for introducing a molecule of interest into a cell is an in vitro method. In an embodiment, the method for introducing a molecule of interest into a cell is an in vivo method. According to the present disclosure, provided may be a molecule of interest, a lipid mixture, a non-ionic lipid, a cationic lipid, an anionic lipid, a combination of a cationic lipid and an anionic lipid, a first complex, an extracellular vesicle (particularly an exosome), a second complex, or a composition comprising any of these, for use in these methods.

In all embodiments, the extracellular vesicle preferably comprises an exosome or is an exosome. In an embodiment, the extracellular vesicle comprises microvesicles or is an exosome. In an embodiment, the extracellular vesicle comprises exosomes and microvesicles. The ratio of exosomes to microvesicles is not particularly limited, but is, for example, 0:100, 10:90, 20:80, 30:70, 40:60, 50:50, 60:40, 30:70, 20:80, 10:90, or 100:0, or a ratio between any two of the above ratios.

Exosomes can be obtained by methods well known to those skilled in the art. Exosomes can be obtained from biological samples (e.g., cell culture supernatants) by, for example, ultracentrifugation, size-exclusion chromatography, ultrafiltration, immunological precipitation methods such as immunomagnetic separation, density gradient centrifugation, or a combination of any two or more thereof. When obtaining exosomes from a cell culture supernatant, the culture supernatant is preferably obtained by culturing the cells in a medium that satisfies one or more, or all, conditions selected from the group consisting of serum-free, albumin-free, cytokine-free, and xeno-free. More preferably, the culture supernatant may be obtained by culturing the cells in a chemically defined medium. Ultracentrifugation can be performed on the supernatant containing exosomes after pre-precipitating solids such as cell debris by centrifugation. In ultrafiltration, exosomes in a sample can be concentrated using a membrane having a specific molecular weight cut-off (MWCO). By passing the sample through the membrane, large particles such as exosomes remain on the membrane and can be collected. In immunological precipitation, exosomes can be separated using antibodies against specific markers on the surface of exosomes (e.g., CD9, CD63, CD81, etc.). For example, exosomes can be easily isolated from a sample by using magnetic beads to which the above antibodies are bound. Density gradient centrifugation is a method of centrifuging a biological sample in a density gradient medium to separate exosomes in the sample as a layer formed depending on their density. Examples of the biological sample include culture supernatants of stem cells such as adipose-derived stem cells and bone marrow-derived stem cells. Examples of the biological sample include culture supernatants of one or more mesenchymal stem cells selected from the group consisting of adipose-derived mesenchymal stem cells, bone marrow-derived mesenchymal stem cells, umbilical cord-derived mesenchymal stem cells, and umbilical cord blood-derived mesenchymal stem cells. Examples of the biological sample also include, for example, milk, preferably raw milk, and particularly raw bovine milk.

Microvesicles can also be obtained basically in the same manner as described above, but it is known that conditions different from those for exosomes can be applied. The differences in the separation of microvesicles and exosomes are mainly based on size, density, and surface markers. However, utilizing these properties, a person skilled in the art would be able to appropriately obtain exosomes and microvesicles separately or as a mixture. In ultracentrifugation, microvesicles can generally be precipitated by centrifuging at a centrifugal force of 10,000 to 20,000 x g for about 20 minutes to 1 hour, whereas exosomes can be centrifuged at a centrifugal force of 100,000 x g or more for several hours. In ultrafiltration, a 100 kDa MWCO filter can generally be used for the separation of exosomes, and a 1000 kDa MWCO filter can be used for the separation of microvesicles. Further, exosomes can be obtained by, for example, an antibody against one or more or all markers selected from the group consisting of CD9, CD63, CD81, ALIX, and TSG101, while microvesicles can be obtained by an antibody against one or more or all markers selected from the group consisting of CD40, selectins, and integrins. Therefore, the obtained extracellular vesicles may express any one or more or all of the above markers.

According to the present disclosure, provided is a composition or a pharmaceutical composition comprising the above complex. The composition or pharmaceutical composition comprising the above complex may further contain a pharmaceutically acceptable additive. Examples of the pharmaceutically acceptable additive include solvents such as water, pH adjusters, osmotic pressure adjusters, thickeners, dispersants, or salts. The composition comprising the above complex may further contain a biologically acceptable additive in addition to the complex. Examples of the biologically acceptable additive include solvents such as water, media or medium components, pH adjusters, osmotic pressure adjusters, thickeners, dispersants, or salts. The composition and the pharmaceutical composition comprising the complex can be used in vitro and in vivo, respectively. The composition comprising the complex may be a cosmetic composition or a dermatological composition. The composition and the pharmaceutical composition may preferably be a liquid preparation. The components contained in the composition (particularly the pharmaceutical composition) to be administered into the body are of a pharmaceutically acceptable type and/or amount.

### EXAMPLES

### 1A. Fusion of Lipid-Protein Complex and Exosome

Exosomes were obtained by purifying Suit2 cell culture supernatant by the PS affinity method (FUJIFILM Wako Pure Chemical). Using a 10 cm dish, cells were cultured in 5 mL of serum-free medium for 24 hours, and then the cell supernatant was collected. Exosomes were purified in accordance with the protocol of the MagCapture(TM) Exosome Isolation Kit PS Ver. 2. The purified exosomes were aliquoted and stored at -30 degrees C until use.

The lipid-protein complex was prepared as follows. As the lipid, an ionic lipid was used here because the production process is simple. 1,2-Dimyristoyl-sn-glycero-3-ethylphosphatidylcholine (EDMPC⁺) was used as the cationic lipid (WO2021/192861, which is incorporated herein by reference in its entirety), and linoleic acid was used as the anionic lipid. The cation and anion were mixed at a 1:1 molar ratio. The lipid mixture exhibited properties of an ionic liquid (IL). A lipid ethanol solution was obtained by diluting with ethanol to the above concentration. An antibody was used as the protein. 10 µL of a Milli-Q aqueous solution (100 ng/µL) of allophycocyanin (APC)-modified anti-hIL-2 antibody (BioLegend, 500310) or APC-modified hCD45 antibody (BioLegend, 304012) and 1 µL of a lipid ethanol solution (5 µg/µL) were added to a 1.5 mL tube and vortex mixed for 15 seconds or more. The tube was then left standing at room temperature for 15 minutes or more to form complex particles (first complex) of the lipid and APC-modified anti-hIL-2 antibody and the lipid and APC-modified anti-hCD45 antibody, thereby obtaining first complexes containing the respective antibodies.

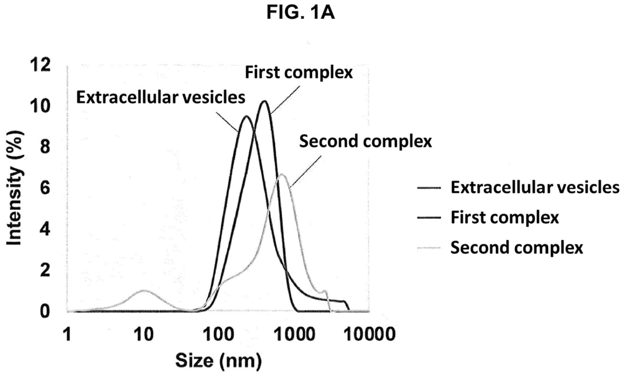

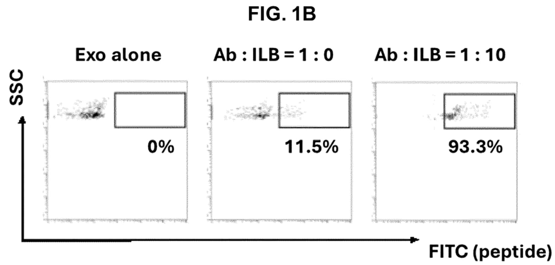

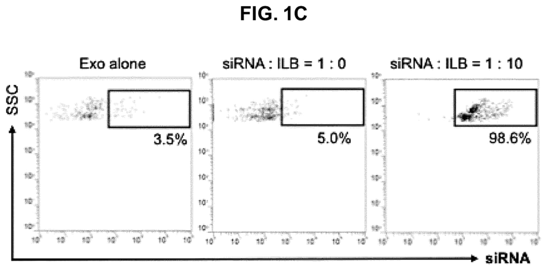

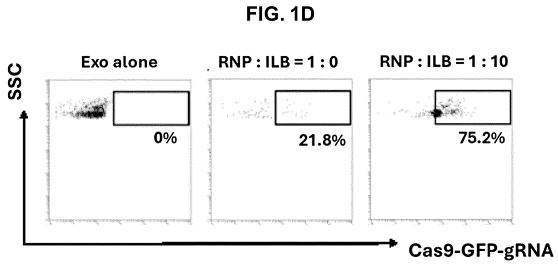

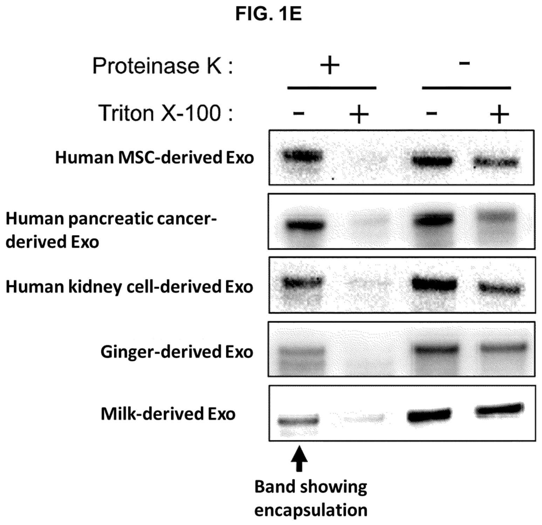

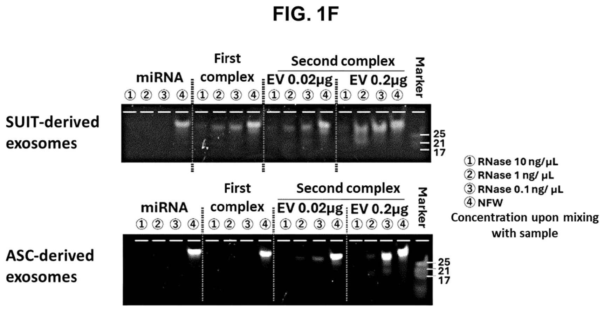

Subsequently, 11 µL of the obtained lipid-protein complex and 10 µL of an exosome aqueous solution (1.0 x 10⁸ particles/10 µL) were vortex mixed, and the mixture was left standing at room temperature for 1 hour or more to obtain lipid-protein-exosome hybrid particles (second complexes, respectively). The particle size distributions of the exosomes and the complex particles were examined by dynamic light scattering (FIG. 1). As shown in FIG. 1, the peak of the particle size distribution shifted to the right. This means that the particle size of the obtained particles became larger, suggesting that the exosomes and the lipid-protein complex further fused to form particles (hereinafter referred to as "hybrid particles").

### 1B. Loading Efficiency of Active Ingredients onto Exosomes Exosomes Used

Exosomes derived from cultured cells were obtained by concentrating the culture supernatants of SUIT-2 cells, human adipose-derived stem cells, and 293 cells by ultrafiltration, followed by purification by size-exclusion chromatography. The cells were cultured in 5 mL of a serum-free medium using a 10-cm dish for 72 hours, and then the cell supernatant was collected. The obtained supernatant was concentrated 10-fold using Amicon Ultra-4 100k (Merck Millipore), and exosomes were purified according to the protocol for qEV columns (IZON). The purified exosomes were aliquoted and stored at - 30°C until use.

Ginger-derived exosomes were purified based on a juice obtained by grating commercially available ginger (from Fukuoka Prefecture) with a hand blender and squeezing it using a non-woven fabric. Milk-derived exosomes were purified based on whey obtained from commercially available Ito Monogatari (Itoshima Milk Plant Co., Ltd.) by concentrating it 10-fold with Amicon Ultra-4 100k (Merck Millipore) and following the protocol for a qEV column (IZON). Cases where other purification kits were used are described individually.

### Measurement of Loading Efficiency of Agents onto Exosomes Using Flow Cytometry

### Reagents

An N-terminally FITC-labeled peptide (SEQ ID NO: 1: KKKSVYDFFVWL) was custom synthesized by Biologica. As siRNA, BLOCK-iT(TM) Fluorescent Oligo, for electroporation was used. Cas9 Nuclease GFP NLS Protein and TrueGuide(TM) sgRNA Positive Control, HPRT1 (human) were purchased from Cosmo Bio and Thermo Fisher Scientific, respectively, and mixed to form RNP (Cas9-gRNA complex). 1,2-Dimyristoyl-sn-glycero-3-ethylphosphatidylcholine (EDMPC⁺) was used as the cationic lipid, and linoleic acid was used as the anionic lipid. The cation and anion were mixed at a 1:1 molar ratio in ethanol.

### Experiment

An aqueous peptide solution (containing 200 ng of FITC-labeled peptide) and a lipid ethanol (ILB) solution (containing 0 or 2 µg of lipid) were vortex mixed and left standing for 15 minutes to form the first complex. Furthermore, SUIT-2 cell-derived exosomes (containing 20 ng as protein) were added, vortex mixed, and left standing for 1 hour to form the second complex (50 µL). The obtained second complex was measured using a flow cytometer, and the ratio of peptide-loaded exosome particles to total exosomes was calculated.

As shown in FIG. 1B, when peptide and exosomes were directly mixed, only about 11.5% of exosomes were loaded with peptide (see middle panel). In contrast, using the scheme in which the peptide and ILB were first mixed to form the first complex, followed by mixing the first complex with exosomes, 93.3% of exosomes were loaded with peptide (see right panel of FIG. 1B).

### (2) Loading Efficiency of Nucleic Acids

### Experiment

An aqueous siRNA solution (containing 200 ng of FITC-labeled siRNA) and a lipid ethanol solution (containing 0 or 2 µg of lipid) were vortex mixed and left standing for 15 minutes to form the first complex. Furthermore, SUIT-2 cell-derived exosomes (containing 20 ng as protein) were added, vortex mixed, and left standing for 1 hour to form the second complex (50 µL). The obtained second complex was measured using a flow cytometer, and the ratio of siRNA-loaded exosome particles to total exosomes was calculated.

As shown in FIG. 1C, only when siRNA and ILB were mixed to form the first complex and then the first complex and exosomes were mixed, nucleic acid was loaded onto substantially all exosomes, with a loading efficiency of 98.6%.

### (3) Loading Efficiency of RNA-Protein Complex (RNP)

An aqueous RNP solution (containing 200 ng of FITC-labeled RNP) and a lipid ethanol solution (containing 0 or 2 µg of lipid) were vortex mixed and left standing for 15 minutes to form the first complex. A Cas9-gRNA complex was used as the RNP. GFP was linked to Cas9. Furthermore, SUIT-2 cell-derived exosomes (containing 20 ng as protein) were added, vortex mixed, and left standing for 1 hour to form the second complex (50 µL). The obtained second complex was measured using a flow cytometer, and the ratio of RNP-loaded exosome particles was calculated.

As shown in FIG. 1D, only when RNP and ILB were mixed to form the first complex and then the first complex and exosomes were mixed, the majority of exosomes were loaded with RNP, with a loading efficiency of 75.2%.

Thus, regardless of whether the molecule was a protein, peptide, nucleic acid, or RNP, components could be very efficiently introduced into exosomes by forming a complex with lipid and then forming a complex with exosomes.

### 1C. Verification of Completion of Encapsulation of Various Components into Exosomes

### (1) Antibodies

### Reagents

Anti-mouse IL-17A-InVivo was purchased from Selleck. Triton X-100 was purchased from Thermo Fisher Scientific. Proteinase K solution (for molecular biology) was purchased from Kanto Chemical. Trans-Blot Turbo Transfer Pack PVDF (Mini) was purchased from Bio-Rad, and Anti-Mouse IgG, HRP-Linked Whole Ab Sheep and ECL Prime Western Blotting Detection Reagent were purchased from Cytiva. Blocking One solution was purchased from Nacalai Tesque.

### Experiment

An antibody aqueous solution (containing 200 ng of antibody) and a lipid ethanol solution (containing 2 µg of lipid) were vortex mixed and left standing for 15 minutes to form the first complex. Furthermore, various exosomes (containing 20 ng as protein) were added, vortex mixed, and left standing for 1 hour to form the second complex (50 µL).

Proteinase K (0 or 0.64 µg/mL) 6 µL and Triton X-100 (0 or 1%) 6 µL were added, and the mixture was incubated at 37 degrees C for 60 minutes. 21 µL of 4x sample buffer was added to the obtained enzymatic reaction solution, incubated at 95 degrees C for 5 minutes, and SDS-PAGE was performed. NuPAGE 4-12% Bis-Tris Gel was used as the gel, and electrophoresis was performed at 100 V for 10 minutes, followed by 200 V for 40 minutes. In this experimental system, if the contents are within the exosome, they would not be degraded by Proteinase K in the absence of Triton X-100, but would be degraded by Proteinase K in the presence of Triton X-100.

For transfer to a membrane, the Trans-Blot Turbo Transfer System (Bio-Rad) was used, and the protein was transferred to a PVDF membrane following the instrument protocol, followed by detection using HRP-labeled anti-mouse IgG. Blocking One solution was used for blocking, and PBS-T was used for washing. ECL Prime Western Blotting Detection Reagent was used for the color reaction, and signal detection was performed using ChemiDoc XRS+ (Bio-Rad).

The results of Western blotting are shown in FIG. 1E. As shown in FIG. 1E, it was found that proteins that were not digested in the absence of Triton X-100 were present near the 150 kDa marker. This is because the antibody was protected from enzymatic degradation by being encapsulated in the exosome. The results of FIG. 1E showed that antibodies can be encapsulated in exosomes derived from various sources.

### (2) Nucleic acids

### Reagents

AccuTarget miRNA Housekeeping Positive control (GAPDH, Human/Mouse) was purchased from Cosmo Bio. Exosomes derived from culture supernatant of SUIT-2 cells were purified using MassivEV EV Purification Column PS / MassivEV Purification Buffer Set (FUJIFILM Wako Pure Chemical). RNase A, DNase and protease-free (10 mg/mL) was purchased from Thermo Fisher Scientific, and GelRed Nucleic Acid Gel Stain, 10,000x in Water was purchased from Cosmo Bio.

### Experiment

A miRNA aqueous solution (containing 20 ng of miRNA) and a lipid ethanol solution (containing 0.02 µg of lipid) were vortex mixed and left standing for 15 minutes to form the first complex. Furthermore, various exosomes (containing 0.02 or 0.2 µg as protein) were added, vortex mixed, and left standing for 1 hour to form the second complex (50 µL).

RNase A (0, 0.1, 1, or 10 ng/µL) dissolved in nuclease-free water (6 µL) was added, and the mixture was incubated at 37 degrees C for 30 minutes. 5x Loading Buffer was added to the obtained enzymatic reaction solution, applied to a 15% polyacrylamide gel, and electrophoresis was performed at 20 mA for 40 minutes. The gel was stained with GelRed, and nucleic acid bands were detected using ChemiDoc XRS+.

From the results shown in FIG. 1F, it can be understood that the nucleic acids encapsulated in exosomes were digested in a concentration-dependent manner of the digestive enzyme, and that the degradation decreased when the amount of EVs mixed increased, indicating that the nucleic acids were protected from degradation by the exosomes, i.e., the nucleic acids were encapsulated in the exosomes. It was also shown that encapsulation occurred in both SUIT-2 cell-derived exosomes and MSC-derived exosomes.

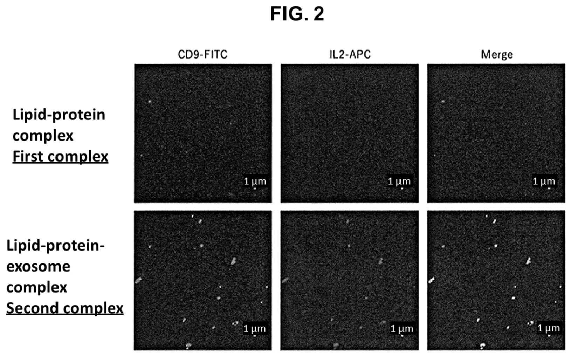

Subsequently, the exosomes in the hybrid particles were stained with a FITC-modified CD9 antibody, and the co-localization of the exosomes and the anti-IL-2 antibody was observed by fluorescence microscopy (FIG. 2). As a result, as shown in FIG. 2, the lipid-anti-IL-2 antibody particles and the exosomes showed co-localization. This suggests that hybrid particles were formed by fusion upon contacting the lipid-anti-IL-2 antibody complex particles with the exosomes, and that the anti-IL-2 antibody was introduced into the exosomes.

### 2A. Introduction of Proteins into Cells by Hybrid Particles

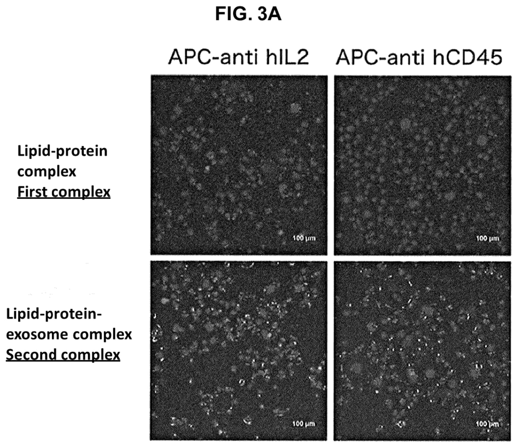

Serum-free medium was added to dispersions of the first complex and the second complex prepared in Section 1 above, followed by gentle mixing and further dropwise addition to cells. After 48 hours of culture and cell fixation, exosomes were stained with a CD9 antibody and observed under a fluorescence microscope. It was shown that exosomes that had formed hybrid particles with the lipid-protein complex were taken up by cells (FIG. 3A). This demonstrated that proteins and protein complexes can pass through cell membranes and be introduced intracellularly by complexing with lipid and further fusing with exosomes.

### 2B-1. Introduction of Proteins into Cells by Hybrid Particles

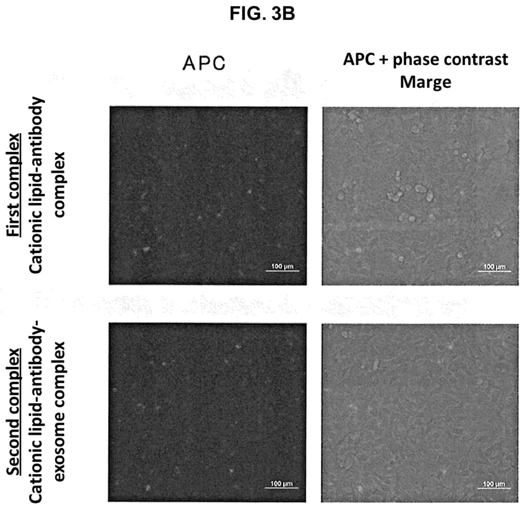

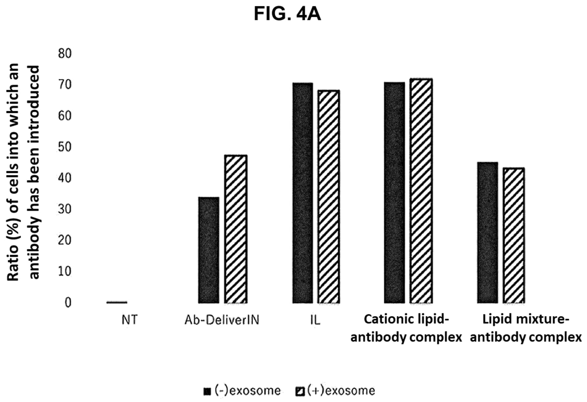

A Milli-Q solution (20 mg/mL) of APC-labeled PD-L1 antibody (BioLegend) and an ethanol solution (6 mg/mL) containing the above liquid lipid composition (IL) were added to a 1.5 mL tube at 10 µL and 1 µL, respectively, and vortex mixed for 15 seconds or more. As the lipid, EDMPC was used as the cationic lipid and linoleic acid was used as the anionic lipid. Specifically, the lipids used were (i) an ionic liquid lipid composition (IL) comprising EDMPC⁺ and linoleic acid, (ii) EDMPC⁺ chloride salt (EDMPC⁺ Cl⁻) (cationic lipid), and (iii) a mixture of EDMPC⁺ and linoleic acid (lipid mixture). (i) was prepared by mixing EDMPC⁺ Cl⁻ and linoleic acid in chloroform, followed by removal of chloroform and HCl by lyophilization, and (iii) was a simple mixture of EDMPC⁺ Cl⁻ and linoleic acid. The mixture was then left standing at room temperature for 15 minutes or more, whereby complex particles of the lipid and PD-L1 antibody were formed. Next, 10 µL of an exosome aqueous solution (1.0 x 10⁸ particles/10 µL) was added to 11 µL of the above lipid-antibody complex, vortex mixed, and left standing at room temperature for 1 hour or more. The above hybrid particles were added to Suit2 pancreatic cancer cells and cultured for 48 hours. APC fluorescence was observed by microscopy. In addition, the proportion of cells into which the antibody was introduced was measured using a flow cytometer. The results are shown in FIG. 3B. As shown in FIG. 3B, the second complex prepared by associating the first complex of a single type of lipid and protein complex with exosomes was also able to effectively introduce antibody molecules intracellularly. Furthermore, as shown in FIG. 4A, both first and second complexes were effectively introduced intracellularly regardless of which lipid was used. The fact that the first complex (cationic lipid-antibody complex) using EDMPC⁺ Cl⁻ was also able to effectively transfer antibodies into cells suggests that the first complex does not need to contain components other than cationic lipids such as anionic lipids or neutral lipids (particularly lipids other than cationic lipids). The first complex (lipid mixture-antibody complex) using EDMPC⁺ Cl⁻ and linoleic acid was also able to effectively transfer antibodies into cells.

### 2B-2. Formation of First Complex Comprising Antibody and Lipid and Introduction of Antibody into Cells by the Complex

APC-labeled anti-human CD274 (BD-H1, PD-L1) Clone: 29E.2A3 was purchased from BioLegend. The antibody was diluted to 10 ng/µL with water. EDMPC Cl salt was dissolved in ethanol to 2.5 mM to obtain lipid solution (i). In addition, EDMPC Cl salt and linoleic acid were mixed in ethanol at 2.5 mM each to obtain lipid solution (ii). 50 µL of antibody solution and 5 µL of lipid solution (i) or (ii) were added to a 1.5 mL tube, stirred by vortex for 60 seconds, and left standing for 15 minutes or more to obtain dispersions containing first complex (i) or (ii), respectively.

To 55 µL of the obtained dispersion containing first complex (i) or (ii), 5 µL of milk exosome solution (protein concentration 0.1 mg/mL) was added, stirred by vortex for 60 seconds, and left standing for 1 hour or more to obtain second complex (i) or (ii).

### Cell Experiment

A suspension of Suit2 cells (3 x 10⁴/200 µL) was prepared and dispensed at 200 µL per well of a multi-well plate. After overnight incubation at 37 degrees C, it was confirmed the next day that cells covered approximately 80-90% of the bottom surface. All medium in the dish was aspirated, and 200 µL of FBS-free medium was added. 55 µL/well of dispersion containing first complex (i) or (ii) or second complex (i) or (ii) was added. After incubation at 37 degrees C for 48 hours, nuclei were stained with Hoechst 33342, and observation was performed under a fluorescence microscope.

### Results

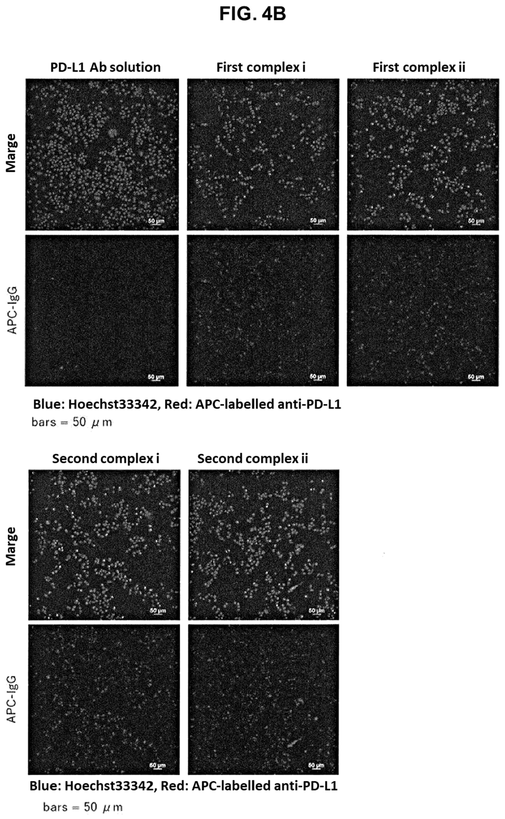

In the example where cells were simply contacted with IgG (PDL1 Ab aqueous solution), almost no fluorescence derived from APC was confirmed in cells, whereas fluorescence derived from APC was confirmed intracellularly in cells administered with either the first complex or the second complex (FIG. 4B). This suggested that antibody introduction into cells is promoted by the antibody taking a complex with anionic lipid or with anionic lipid and cationic lipid. Furthermore, antibody introduction into cells was further promoted by fusing the complex with exosomes. These results were similar when the mixing ratio of anionic lipid and cationic lipid was varied.

### 2C. Introduction of Proteins into Cells by Hybrid Particles

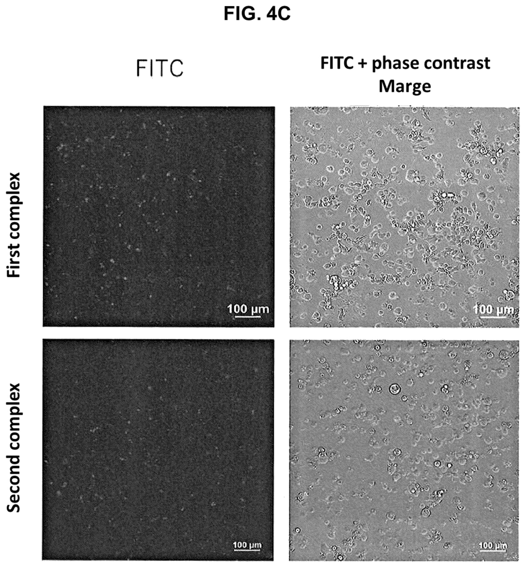

A Milli-Q solution (1 mg/mL) of N-terminally FITC-labeled peptide (SEQ ID NO: 1: KKKSVYDFFVWL) and an ethanol solution (10 mg/mL) containing the above liquid lipid composition (IL) were added to a 1.5 mL tube at 10 µL and 1 µL, respectively, and vortex mixed for 15 seconds or more. The mixture was then left standing at room temperature for 15 minutes or more, whereby complex particles (first complex) of the lipid and peptide were formed. Next, 10 µL of an exosome aqueous solution (1.0 x 10⁸ particles/10 µL) was added to 11 µL of the above lipid-peptide complex, vortex mixed, and left standing at room temperature for 1 hour or more to obtain hybrid particles (second complex). The above hybrid particles were added to a culture containing Suit2 pancreatic cancer cells and cultured for 48 hours. FITC fluorescence was observed under a phase-contrast fluorescence microscope, and phase-contrast images of the same field of view were obtained. The results are shown in FIG. 4C. As shown in FIG. 4C, both the first complex and the second complex were effectively delivered intracellularly. Thus, both the first complex and the second complex had the ability to deliver the molecule of interest intracellularly.

### 2D. Introduction of siRNA into Cells by Hybrid Particles Complex Preparation

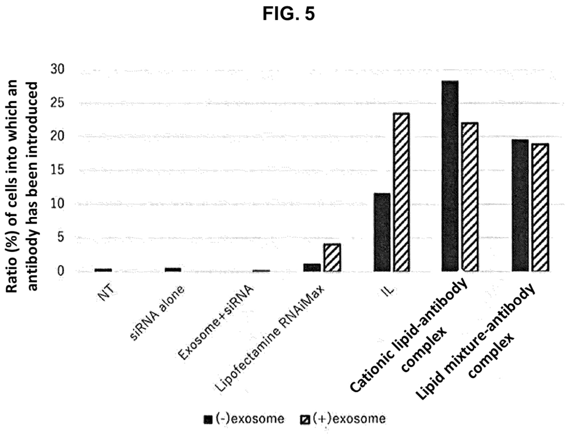

10 µL of an aqueous HiLyte488-siRNA solution (0.2 nmol/mL) and a lipid ethanol solution (6 mg/mL) were added to a 1.5 mL tube at 10 µL and 1 µL, respectively, and vortex mixed for 15 seconds or more. As the siRNA, HiLyte-488-labeled positive control siRNA (Human β-actin) purchased from Nippon Gene was used. As the lipid, an ionic liquid lipid composition (IL) of EDMPC+ and linoleic acid, EDMPC+ chloride salt, and a mixture of EDMPC+ and linoleic acid were used. The mixture was then left standing at room temperature for 15 minutes or more, whereby complex particles of the lipid and siRNA were formed (first complex). Next, 10 µL of an exosome aqueous solution (1.0 x 10⁸ particles/10 µL) was added to 11 µL of the above lipid-siRNA complex, vortex mixed, and left standing at room temperature for 1 hour or more. The above hybrid particles were added to Suit2 pancreatic cancer cells and cultured for 48 hours. HiLyte488 fluorescence was observed by microscopy. In addition, the proportion of cells into which the antibody was introduced was measured using a flow cytometer. The results are shown in FIG. 5. As shown in FIG. 5, both the first complex and the second complex were effectively introduced intracellularly regardless of which lipid was used. The fact that the first complex using EDMPC+ Cl- was also able to effectively transfer antibodies into cells suggests that the first complex does not need to contain components other than cationic lipids such as anionic lipids or neutral lipids (particularly lipids other than cationic lipids). In this experiment as well, both the first complex and the second complex had the ability to deliver the molecule of interest intracellularly.

### 3A. Investigation of Intracellular MMP-7 (Enzyme) Inhibition Effect by Antibody

First, an anti-matrix metalloproteinase-7 antibody (anti-MMP-7 antibody) was delivered intracellularly using the first complex, and the intracellular MMP-7 (enzyme) inhibition effect was investigated.

### Materials

Anti-MMP-7 antibody was purchased from Proteintech. As a positive control, Ab-DeliverIN, an antibody transfection reagent, was purchased from OZ Biosciences and used. Small extracellular vesicles (sEV) of Suit2 cells were obtained by purifying the cell culture supernatant using the PS affinity kit. The amount of protein contained in the obtained sEV dispersion was quantified by the BCA method, and the number of particles was measured using NanoSight.

### Complex Preparation

The antibody was diluted to 10 ng/µL with water. EDMPC Cl salt and linoleic acid were mixed in ethanol at 2.5 mM each to obtain a lipid solution. 50 µL of antibody solution and 5 µL of lipid solution were added to a 1.5 mL tube, stirred by vortex for 60 seconds, and left standing for 15 minutes or more to obtain a dispersion containing the first complex. sEV was diluted to 0.1 µg/mL, and 5 µL of the sEV dispersion was added to 55 µL of the dispersion containing the first complex, stirred by vortex for 60 seconds, and left standing for 1 hour or more to obtain the second complex.

### Cell Experiment

Matrigel (20 µg/mL) was added to a 96-well plate for cell invasion assay, and the pores (phi 8 µmeter) of the polycarbonate membrane in the chamber were filled with Matrigel. A suspension of Suit2 cells was adjusted to 1 x 10⁵/mL and dispensed at 2.5 x 10⁴/well into the upper chamber. After overnight incubation at 37 degrees C, it was confirmed the next day that cells covered 90% or more of the bottom surface.

All medium in the dish was aspirated, and FBS-free medium was added to the lower chamber at 750 µL and to the upper chamber at 195 µL. Subsequently, a lipid complex dispersion containing 500 ng of antibody was administered to the cells in the upper chamber (antibody dose was 500 ng/well). As a positive control, a complex of Ab-DeliverIN and antibody was administered, and as a negative control, an antibody aqueous solution was administered (antibody dose was 500 ng/well). Cells to which 50 µL of medium without antibody was added served as Non-Treat. After incubation at 37 degrees C for 48 hours, the number of cells in the upper chamber was counted to calculate the cell proliferation rate. Subsequently, cells remaining on the upper part of the polycarbonate membrane were removed with a cotton swab, and cells that had invaded to the lower part of the membrane were stained with H&E. Cells that had invaded to the lower part of the polycarbonate membrane were observed by microscopy. The number of cells present per microscopic field of view was counted, and the cell number was calculated as a percentage where the number of invading cells in Non-Treat was set to 100.

### Results

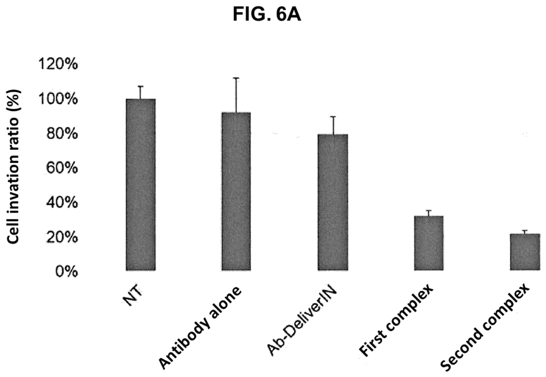

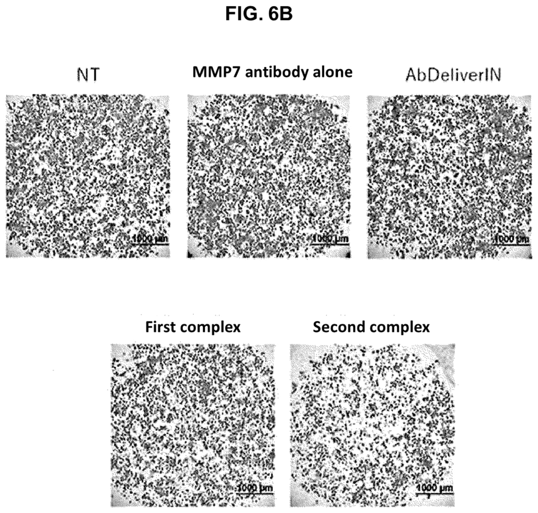

No significant difference in cell proliferation rate was observed among cells administered with any of the formulations, indicating that the formulations did not exhibit cytotoxicity. On the other hand, cells administered with anti-MMP-7 antibody alone did not show a cell invasion-suppressing effect, whereas cell invasion was suppressed in cells administered with the first complex and the second complex (see FIGS. 6A and 6B). From these results, it is considered that complexation with lipid promoted the uptake of anti-MMP-7 antibody into the cytoplasm of Suit2 cells, thereby suppressing cell invasion. It was also demonstrated that the antibody maintained its binding properties to the antigen even after complexation with lipids.

Note that a formulation comprising a solid complex of an antigen and a lipid and an oily base, in which the solid complex is dispersed in the oily base, enhances the skin permeability of the antigen (see Japanese Patent No. 5752343). An antibody can be mixed with a lipid in cyclohexane and emulsified, followed by lyophilization to form a solid complex comprising the anti-VEGF antibody and the lipid, which can then be dispersed in an oily base (isopropyl myristate), thereby obtaining a formulation in which the solid complex comprising the antibody and the lipid is dispersed in the oily base (the hydrodynamic diameter was approximately 100 nm). Using aflibercept instead of the anti-VEGF antibody, a formulation was obtained in which a solid complex comprising aflibercept and a lipid was dispersed in an oily base. Furthermore, this formulation was able to sustain the release of a functional antibody or protein by intravitreal administration or eye drops. Furthermore, this formulation exhibited skin permeability, and the permeated antibody possessed binding affinity for its antigen. Thus, a complex of an antigen and a lipid can be formed into a solid complex and can also be used by being dispersed in an oily base.

### 3B. Investigation of Intracellular MMP-7 (Enzyme) Inhibition Effect

Next, the anti-MMP-7 antibody was delivered intracellularly using the second complex, and the intracellular MMP-7 (enzyme) inhibition effect was investigated. Specifically, 5 mg of anti-MMP-7 antibody (mouse, monoclonal) was dissolved in 200 µL of Milli-Q water. 30 mg of lipid was dissolved in 20 µL of ethanol. As the lipid, a 1:1 ionic liquid lipid composition (IL) of EDMPC⁺ and linoleic acid, EDMPC⁺ chloride salt (cationic lipid), and a mixture of EDMPC⁺ and linoleic acid (lipid mixture) were used. 20 µL of antibody aqueous solution and 2 µL of lipid ethanol solution were mixed, vortexed for 60 seconds, and left standing for 15 minutes to obtain a lipid-protein complex sample (first complex) (22 µL). To 22 µL of the lipid-antibody complex sample, 10 µL of exosome dispersion (containing 1 x 10⁹ particles, 200 ng of exosomes) was added, vortex mixed, and left standing for 1 hour to obtain lipid-antibody-exosome hybrid particles (second complex) (32 µL).

Matrigel PBS dispersion was added at 20 µg/well to the inner cups of a 24-well plate for cell migration assay. After 2 hours, PBS was removed from the wells, and 250 µL/well of serum-free medium was added to swell the gel. After 30 minutes, Suit2 pancreatic cancer cells were seeded on the Matrigel at 2.5 x 10⁴/well. After 24 hours, the medium was replaced with serum-free MEM medium, and the above-prepared samples were added in their entirety. After 72 hours of culture, the number of cells remaining on the Matrigel was counted from phase-contrast observation images to calculate the cell proliferation rate. In addition, cells that had migrated to the well below the Matrigel were fixed with ethanol, stained with H&E, and counted.

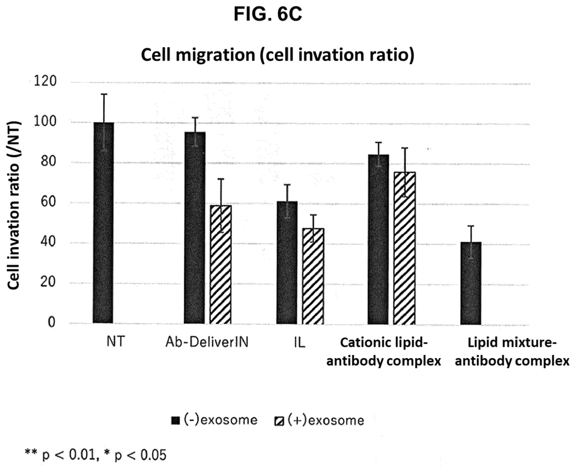

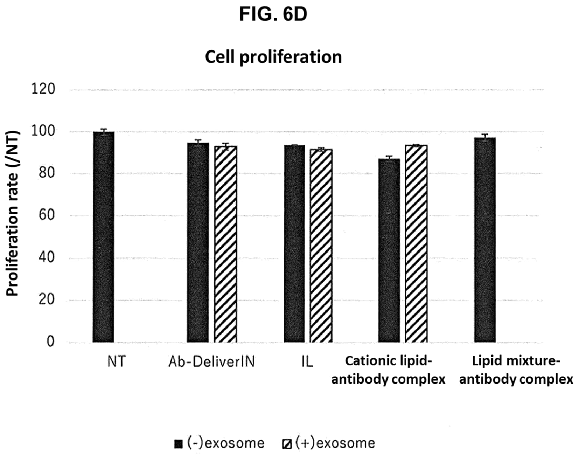

The results are shown in FIGS. 6C and 6D. As shown in FIG. 6D, the intracellular introduction of MMP-7 had almost no effect on cell proliferation. Furthermore, as shown in FIG. 6C, the first complex inhibited cell migration, and the second complex markedly inhibited cell migration. From these results, it was demonstrated that lipid-antibody-exosome hybrid particles can inhibit cell migration by inhibiting cellular functions (particularly the intracellular function of MMP-7) without inhibiting cell proliferation. The second complex showed a higher cell migration inhibition effect than the first complex, and it was considered that lipid-antibody-exosome hybrid particles have higher efficiency for introducing functional antibodies into cells. Comparing the activities of the first complex and the second complex, both the first complex and the second complex effectively inhibited cell migration, but the second complex showed a stronger cell migration inhibition effect. Extracellular vesicles (particularly exosomes) are considered to contribute to intercellular communication and can efficiently transfer internal substances to recipient cells in a functional state, which is considered to be the reason for the increased inhibition effect. The fact that the first complex using EDMPC⁺ Cl⁻ was also able to effectively transfer antibodies into cells suggests that the first complex does not need to contain components other than cationic lipids such as anionic lipids or neutral lipids (particularly lipids other than cationic lipids). In addition, it was demonstrated that the first complex and the second complex have the ability to deliver large protein complexes such as antibodies (approximately 150 kDa) intracellularly.

### 4. Cell Introduction by Nucleic Acid-Loaded Hybrid Particles

Alexa647-labeled antisense oligonucleotide (ASO)-loaded lipid-exosome hybrid particles were prepared by the following method. 10 µL of a Milli-Q aqueous solution (100 ng/µL) of ASO and 1 µL of an ethanol solution (5 µg/µL) of the liquid lipid composition were added to a 1.5 mL tube and vortex mixed for 15 seconds or more. The mixture was then left standing at room temperature for 15 minutes or more to form complex particles of ASO and lipid.

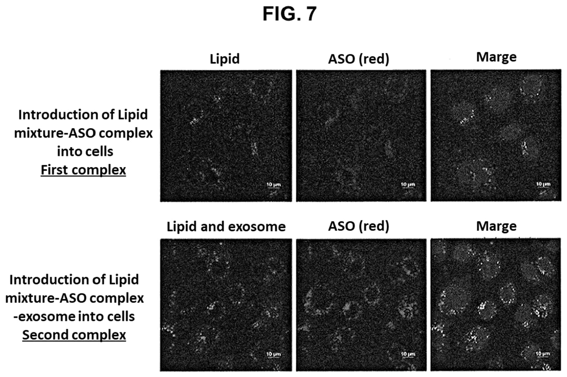

Subsequently, the obtained ASO-lipid complex particles were mixed with an exosome aqueous solution to fuse the ASO-lipid complex particles with exosomes and left standing at room temperature for 1 hour or more to obtain lipid-ASO-exosome hybrid particles. Serum-free medium was added to the obtained hybrid particle dispersion, followed by gentle mixing and further dropwise addition to cells. After 48 hours of culture and cell fixation, exosomes were stained with Alexa488-modified CD-9 antibody and observed under a fluorescence microscope (FIG. 7). As shown in FIG. 7, in the group treated with hybrid particles, massive transfer of ASO into cells was observed. This suggested that lipid-exosome hybrid particles efficiently promote intracellular introduction of ASO.

### 5. Tissue Distribution of Hybrid Particles

### Reagents

14:0 EPC Cl salt (Avanti Polar Lipids) and linoleic acid (Sigma-Aldrich) were purchased from Merck. EPC is an abbreviation for 1,2-dimyristoyl-sn-glycero-3-ethylphosphocholine. Normal human IgG (143-09501) was purchased from FUJIFILM Wako Pure Chemical. Exosomes were purified from commercially available milk-derived exosomes (Cosmo Bio) using the PS Affinity Kit (FUJIFILM Wako Pure Chemical). Normal human IgG is a purified product obtained by purifying total IgG contained in human serum by Protein A chromatography. 14:0 EPC (Cl salt) and linoleic acid were dissolved at equimolar amounts in chloroform to obtain a liquid composition, which was stirred and then lyophilized to obtain a lipid base.

### Particle Preparation

The lipid base-antibody complex (first complex) was prepared as follows. An IgG aqueous solution (0.5 mg/mL) and a lipid base ethanol solution (50 mg/mL) were added to a 1.5 mL tube at 400 µL and 40 µL, respectively, and vortex mixed for 60 seconds or more. The mixture was then left standing at room temperature for 15 minutes or more to form particles (first complex). Subsequently, the obtained first complex (440 µL) and the above purified exosome solution (protein concentration 333 µg/mL, 60 µL) were mixed and left standing at room temperature for 1 hour or more to obtain the second complex (500 µL).

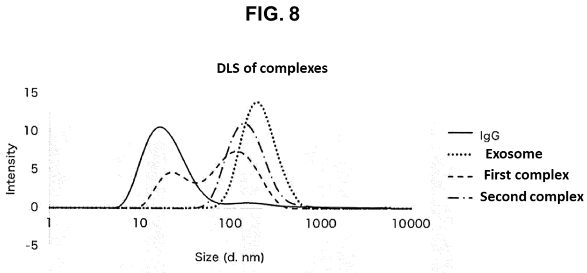

The particle size distributions of the commercially available extracellular vesicles, the first complex, and the second complex were investigated by dynamic light scattering using a Zetasizer Pro (Malvern). The results are shown in FIG. 8 and Table 1. As shown in FIG. 8, in the second complex, exosomes bound to the first complex, and the particle size became larger.

**[Table 1]**

| Table 1: Physical Characteristics of Each Particle | | | |
|---|---|---|---|
| Sample | Average Particle Size (nm) | PdI | Zeta Potential |
| IgG | 18.5 | 0.254 | 15 |
| Exosome | 193.6 | 0.189 | -25.1 |
| First Complex | 126.5, 26.2 | 0.440 | 32.6 |
| Second Complex | 163.3 | 0.377 | 35.8 |

### Tissue Observation

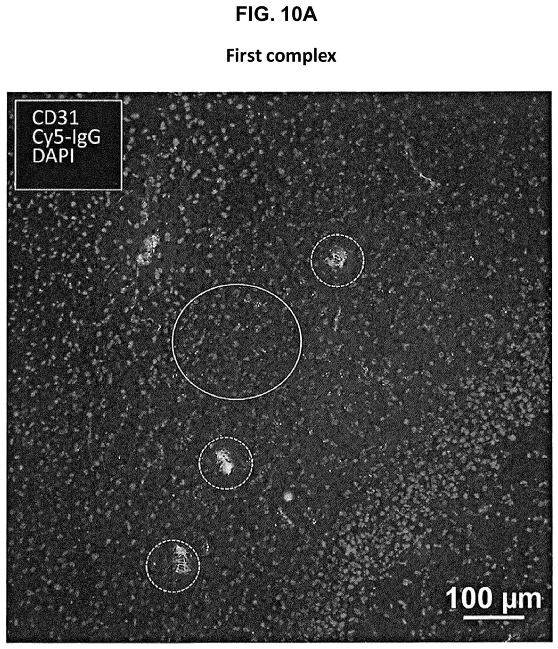

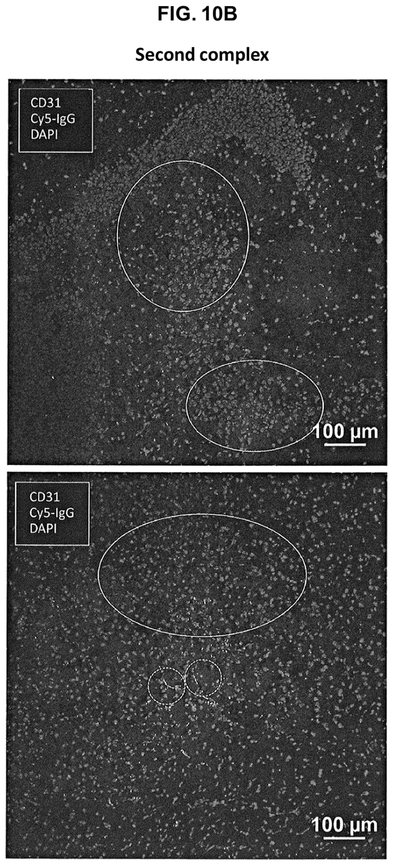

The first complex and the second complex were also prepared using Cy5-labeled normal human IgG in the same manner as described above and administered via the mouse tail vein. After 72 hours, the brain was harvested and frozen sections were prepared. Then, to confirm the localization of IgG (Cy5), the positional relationship between red particles (IgG) and nuclei was investigated. As a result, as shown in FIG. 10A, the first complex tended to be located at a distance from nuclei in brain tissue, whereas as shown in FIG. 10B, the second complex tended to be located in close proximity to nuclei. This suggests that, while the first complex delivers IgG to brain tissue, its efficiency for intracellular delivery is low, whereas the second complex has higher efficiency for delivering IgG into brain cells. That is, it was demonstrated that further complexation of the first complex with exosomes improves the efficiency of intracellular introduction of the complex in brain tissue. Furthermore, both the first complex and the second complex are suitable for substance delivery to brain tissue and into brain cells, but the lipid-antibody complex (first complex) is relatively more advantageous for delivering antibodies to brain tissue, and the lipid-antibody-exosome complex (second complex) is relatively more advantageous for delivering antibodies into brain cells.

### 6. Localization of Antibody in Mouse Brain After Tail Vein Injection Reagents

NeuroTrace(TM) 500/525 Green Fluorescent Nissl Stain - Solution in DMSO was purchased from Thermo Fisher Scientific. Iba1/AIF-1 (E4O4W) XP(TM) Rabbit mAb (Alexa Fluor(TM) 488 Conjugate) was purchased from Cell Signaling. ProLong(TM) Glass Antifade Mountant with NucBlue(TM) Stain was purchased from Thermo Fisher Scientific.

### Experiment

100 µL of an aqueous solution of Cy5-labeled human IgG (containing 100 µg of antibody) and 10 µL of the above lipid ethanol solution (containing 1 mg of lipid) were vortex mixed and left standing for 15 minutes to form the first complex. Furthermore, 100 µL of commercially available milk-derived exosomes (containing 10 µg as protein) was added, vortex mixed, and left standing for 1 hour to form the second complex (210 µL).

200 µL was administered via the mouse tail vein, and after 48 hours, perfusion was performed using PBS followed by 4% paraformaldehyde (PFA). After harvesting the brain tissue, it was immersed in 4% PFA and fixed at room temperature for 3 hours. After immersion in PBS at room temperature for 1 hour, the tissue was immersed in 30% sucrose solution and left at 4 degrees C overnight.

The sunken tissue was removed, embedded in OCT compound, and 10 µmeter frozen sections were prepared using a cryostat. After drying the sections, they were washed with PBS and blocked with Blocking One (blocking solution) containing 0.1% Triton X-100 at room temperature for 1 hour. For Nissl staining, Nissl staining solution diluted 1,000-fold with blocking solution was used and reacted at 4 degrees C overnight. For staining with Iba-1 antibody, the antibody diluted 500-fold with blocking solution was reacted at 4 degrees C overnight. After washing with PBS, sections were mounted using mounting medium containing nuclear staining dye and observed under a fluorescence microscope.

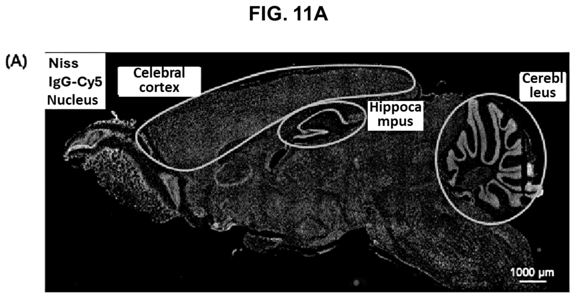

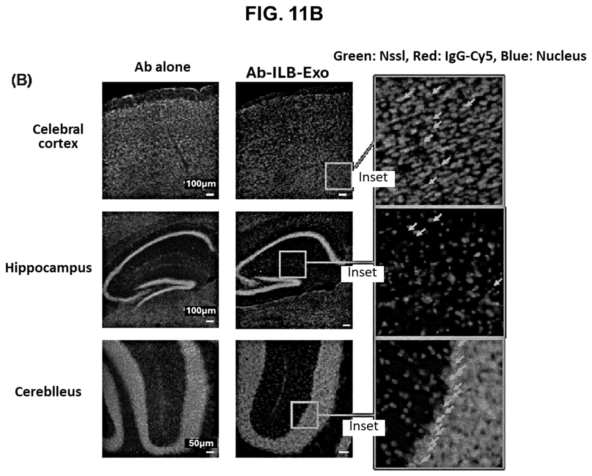

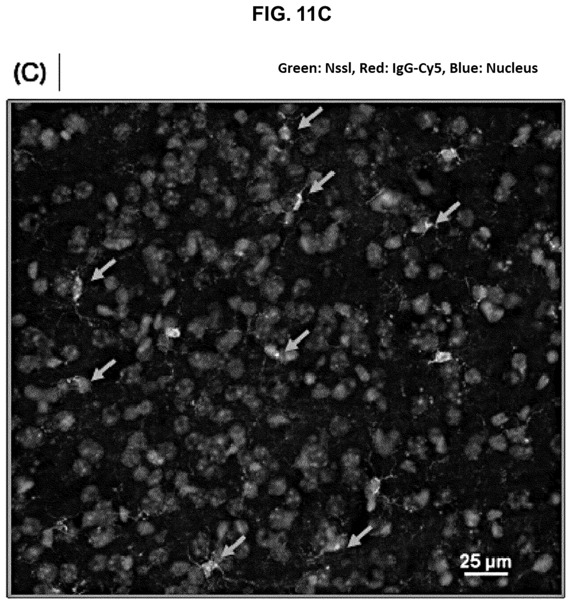

The results are shown in FIGS. 11A to 11C. As shown in FIG. 11A, IgG was delivered throughout the brain. In particular, uptake of IgG was confirmed in the deep cerebral cortex, the central hippocampus, and near Purkinje cells of the cerebellum (FIG. 11A). Upon detailed observation of the cerebral cortex, hippocampus, and cerebellum, as shown in FIG. 11B, fluorescence derived from IgG-Cy5 was observed intracellularly in each tissue. The fluorescent areas are indicated by arrows in FIG. 11B. Furthermore, as shown in FIG. 11C, uptake of IgG by microglia cells in the cerebral cortex was also confirmed (see arrows).

### 7. Antibody Loading onto Lipid Nanoparticles

### Preparation of Lipid Nanoparticles

LipidLaunch LNP-0315 Exploration Kit was purchased from Cayman Chemical Company, and particles were prepared according to the kit protocol without RNA. The lipid concentration and lipid mixing speed during particle preparation followed the kit protocol, and particles were prepared using a microfluidic device, followed by dialysis for 18 hours using a 3.5k MWCO dialysis membrane, and the solvent in which the lipid nanoparticles were dispersed was replaced with 1x PBS.

### Antibody Loading

Normal human IgG, whole molecule, purified was purchased from FUJIFILM Wako Pure Chemical Corporation. An antibody 0.1 mg/mL aqueous solution (100 µL) and an ionic lipid 1 mg/mL ethanol solution (10 µL) were vortex mixed and left standing for 15 minutes to prepare the first particles. Subsequently, the lipid nanoparticle 10 mg/mL PBS solution (10 µL) prepared above was added, vortex mixed, and left standing for 1 hour to obtain the second particles. The obtained solution was analyzed by size exclusion chromatography, and the amount of unencapsulated IgG antibody was quantified. The amount of antibody loaded onto lipid nanoparticles was calculated by the following formula: 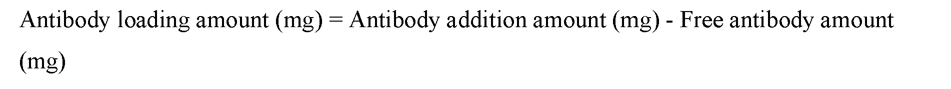 Antibody loading amount (mg) = Antibody addition amount (mg) - Free antibody amount (mg)

**[Table 2]**

| Table 2: Composition of Lipid Nanoparticles and Antibody Loading Amount | | | | | | | |
|---|---|---|---|---|---|---|---|
| Components | Antibody | Ionic Lipid | ALC-0315* | 1, 2-DSPC* | Cholesterol* | ALC-0159* | Antibody Loading Amount |
| Amount Added (mg) | 1 | 1 | 6 | 1 | 3 | 1 | 0.51 |

| | | | | | | | |
|---|---|---|---|---|---|---|---|
| *Names as indicated on the kit | | | | | | | |

### 8. Antibody Loading onto Liposome Particles

### Preparation of Liposome Particles

Mixed lipid Presome ACD-1 was purchased from Nippon Fine Chemical Co., Ltd. Presome ACD-1 was uniformly dispersed in 1x PBS at 60 degrees C, and then passed through a polycarbonate membrane filter with 0.1 µmeter pore diameter 22 times using an extruder to prepare liposome particles.

### Antibody Loading

Normal human IgG, whole molecule, purified was purchased from FUJIFILM Wako Pure Chemical Corporation. An antibody 0.1 mg/mL aqueous solution (100 µL) and an ionic lipid 1 mg/mL ethanol solution (10 µL) were vortex mixed and left standing for 15 minutes to prepare the first particles. Subsequently, the liposome particle 10 mg/mL PBS solution (10 µL) prepared above was added, vortex mixed, and left standing for 1 hour to obtain the second particles. The obtained solution was analyzed by size exclusion chromatography, and the amount of unencapsulated IgG antibody was quantified. The amount of antibody loaded onto liposome particles was calculated by the following formula: 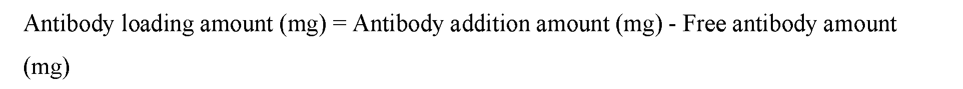 Antibody loading amount (mg) = Antibody addition amount (mg) - Free antibody amount (mg)

**[Table 3]**

| Table 3: Composition and Antibody Loading Amount of Liposome Particles | | | | | | |
|---|---|---|---|---|---|---|
| Component | Antibody | Ionic Lipid | HSPC* | Cholesterol* | MPEG2000-DSPE* | Antibody Loading Amount |
| Amount Added (mg) | 1 | 1 | 6 | 2 | 2 | 0.67 |

| | | | | | | |
|---|---|---|---|---|---|---|
| * Names as indicated on Presome ACD-1 | | | | | | |

## Claims

1. A composition comprising a complex of a molecule of interest, a lipid mixture, and a lipid vesicle.

2. The composition according to claim 1, wherein the lipid mixture and the lipid vesicle form a vesicle, and the molecule of interest is encapsulated in the vesicle.

3. The composition according to claim 1 or 2, wherein the lipid mixture comprises a cationic lipid.

4. The composition according to claim 3, comprising the cationic lipid and an anionic lipid.

5. The composition according to claim 3 or 4, wherein the anionic lipid is represented by R-COO⁻, wherein R represents a substituted or unsubstituted alkyl group, or a substituted or unsubstituted alkenyl group, at least one ethylene group constituting the alkenyl group may be substituted with a vinylene group, and preferably R is a cis-type unsaturated fatty acid.

6. The composition according to any one of claims 3 to 5, wherein the cationic lipid is represented by R-O-P(OR¹)(OR²)(=O), wherein R represents a substituted or unsubstituted alkyl group, or a substituted or unsubstituted alkenyl group, at least one ethylene group constituting the alkenyl group may be substituted with a vinylene group, and preferably R is a cis-type unsaturated fatty acid, R¹ represents an alkyl group substituted with a cationic group, and R² represents a hydrogen atom or a substituted or unsubstituted alkyl group.

7. The composition according to any one of claims 1 to 6, wherein the molecule of interest is a polypeptide.

8. The composition according to any one of claims 1 to 6, wherein the molecule of interest is an antibody or an antigen-binding fragment thereof.

9. The composition according to any one of claims 1 to 6, wherein the molecule of interest is a nucleic acid.

10. The composition according to any one of claims 1 to 9, for delivering the molecule of interest to a cell.

11. The composition according to any one of claims 1 to 10, wherein the complex is obtained by a method comprising: obtaining a complex (first complex) of the molecule of interest and the lipid mixture; and contacting the obtained complex (first complex) with the lipid vesicle to obtain a composite particle (second complex) of the complex and the lipid vesicle, wherein the molecule of interest is encapsulated in the lipid vesicle.

12. A method for introducing a molecule of interest into a lipid vesicle, comprising:
obtaining a complex (first complex) of the molecule of interest and a lipid mixture; and contacting the obtained complex with the lipid vesicle to obtain a composite particle (second complex) of the complex and the lipid vesicle, whereby the molecule of interest is introduced into the composite particle.

13. A method for introducing a molecule of interest into a cell, comprising: obtaining a complex (first complex) of the molecule of interest and a lipid mixture; contacting the obtained complex with a lipid vesicle to obtain a composite particle (second complex) of the complex and the lipid vesicle, whereby the molecule of interest is introduced into the composite particle; and contacting the obtained composite particle with the cell to introduce the molecule of interest into the cell.

14. A dispersion formulation comprising a particle comprising a solid complex of a molecule of interest (preferably a water-soluble molecule) and a lipid mixture (comprising one or more lipidic surfactants) comprising 1,2-dimyristoyl-sn-glycero-3-ethylphosphatidylcholine (EDMPC⁺) or a pharmaceutically acceptable salt thereof, and an oily base, wherein the particle is dispersed in the oily base.

15. The dispersion formulation according to claim 14, wherein the lipid mixture consists of 1,2-dimyristoyl-sn-glycero-3-ethylphosphatidylcholine (EDMPC⁺) or a pharmaceutically acceptable salt thereof (preferably a chloride salt).

16. The dispersion formulation according to claim 14 or 15, wherein the pharmaceutically acceptable salt is a chloride salt of EDMPC⁺ (EDMPC⁺ Cl⁻).

17. The dispersion formulation according to any one of claims 14 to 16, wherein the lipid mixture further comprises an anionic lipid.

18. The dispersion formulation according to claim 17, wherein the anionic lipid comprises any one or more selected from the group consisting of oleic acid (OA), 1,2-dioleoyl-sn-glycero-3-phosphatidic acid (DOPA), 1,2-dioleoyl-sn-glycero-3-phosphoglycerol (DOPG), cardiolipin (CL), 1,2-di-(9Z-octadecenoyl)-sn-glycero-3-phospho-L-serine (DOPS), and phosphatidylinositol (PI).

19. The composition according to any one of claims 1 to 9, for use in delivering the molecule of interest to the brain.

20. The composition according to any one of claims 1 to 9, for use in delivering the molecule of interest into brain cells.

21. An aqueous dispersion solution comprising (i) a complex of a lipid and an antibody and (ii) an aqueous base or an oily base, wherein the complex is dispersed in the base.

22. The aqueous dispersion solution according to claim 21, wherein the lipid comprises or consists of a cationic lipid.

23. The aqueous dispersion solution according to claim 21 or 22, wherein the lipid comprises or consists of 1,2-dimyristoyl-sn-glycero-3-ethylphosphatidylcholine (EDMPC⁺) or a pharmaceutically acceptable salt thereof (preferably a chloride salt).

24. The aqueous dispersion solution according to any one of claims 21 to 23, wherein the lipid further comprises an anionic lipid.
